# EUROPEAN PATENT APPLICATION

(11) **EP 2 522 339 A2**
(43) Date of publication of application: **14.11.2012**
(21) Application number: 12179591.8
(22) Date of filing: 11.03.2008
(51) Int. Cl.: A61K 9/70

(54) **Amorphous drug transdermal systems, manufacturing methods, and stabilization**

(30) Priority: 16.03.2007 US 725159
(62) Divisional of application: 08726714.2
(71) Applicant: MYLAN TECHNOLOGIES, INC., St. Albans, VT 05478 (US)
(72) Inventor: Tang, Jiansheng, South Burlington, VT 05403 (US); Deverich, Joseph, M., Essex Junction, VT 05452 (US); Miller, Kenneth, J., II., St. Albans, VT 05478 (US); Beste, Russell, D., So. Burlington, VT Vermont 05403 (US)
(74) Representative: Eddowes, Simon

(57) **Abstract**

The present invention refers to a transdermal delivery device comprising a backing layer, an adhesive matrix layer comprising a supersaturated concentration of an active agent substantially in amorphous form within the adhesive matrix, and a release liner. The present invention also refers to a method of preparing an adhesive matrix containing at least one supersaturated active agent substantially in amorphous form. Further, the present invention refers to a method to stabilize and a method to reestablish the meta-stable amorphous-drug transdermal system during its manufacturing, storing, shipping and handling process.

## Description

### CROSS-REFERENCE TO RELATED APPLICATIONS

The present application claims the benefit of Application Serial No. 11/725,159, filed March 16, 2007, entitled Amorphous Drug Transdermal Systems, Manufacturing Methods, and Stabilization, the disclosure of which is hereby incorporated herein by reference.

### BACKGROUND OF THE INVENTION

The present invention relates to transdermal drug delivery systems.

The delivery of drugs through the skin provides many advantages. Primarily, it is a comfortable, convenient and non-invasive way of administering drugs. Moreover, such a means of delivery provides for uninterrupted therapy and a higher degree of control over drug concentrations in the blood.

U.S. patent 5,164,190 discloses transdermal administration of hydrophobic drugs via a diffusion mechanism in which the drug is dissolved in a carrier at concentrations between 20% and 80% of saturation concentration. This patent, however, fails to suggest an amorphous transdermal drug delivery system in which the drug is supersaturated and in which the supersaturated portion of the drug is present in an amorphous drug-in-adhesive matrix.

United States Patent No. 4,409,206 discloses a preparation in the form of a polyacrylate film with an amorphous active pharmaceutical ingredient embedded therein. This patent does not, however, disclose a transdermal delivery device or a system containing a supersaturated concentration of an amorphous drug within an adhesive matrix.

United States Publication No. 2005/0064022 describes a device comprising amorphous terazosin. More specifically, the publication discloses a transdermal therapeutic system for the administration of amorphous terazosin to the skin comprising a backing layer, a pressure-sensitive adhesive reservoir layer and/or a matrix layer, and optionally a removable protective layer.

United States Publication No. 2005/0175678 A1 is directed to a polymer matrix suitable for the transdermal administration of rotigotine and a method of preparing the same. The polymer matrix contains a supersaturated amount of a rotigotine base such that the part of the rotigotine that is not dissolved in the matrix polymer is dispersed in the matrix as amorphous particles. The publication further discloses that the matrix may be a component of a system for transdermal administration of rotigotine, wherein the system can have components such as a protective layer, a backing layer, further polymer layers, and/or a membrane which controls release of the rotigotine.

United States Patent No. 6,902,741 is directed to a transdermal system which includes a sex hormone-containing adhesive matrix, containing inclusions of sex hormone in a hydrophilic non-crosslinked polymer. The active substance contained in the inclusions is preferably amorphous to an extent of more than 50% by weight of the active substance. The active substance-containing laminate is characterized in that the active substance inclusions are contained in the adhesive matrix in dissolved or dispersed form.

Various methods of manufacturing transdermal systems in which the drug is supersaturated are known. U.S. Patent Nos. 4,409,206, 4,490,322, 4,797,284, 4,880,633, 5,352,457 5,869,089, 5,906,830, 6,153,216, 6,156,335, and 6,623,763 describe methods of manufacturing transdermal systems. U.S. Patent No. 4,490,332 discloses a method of manufacturing a polyacrylate film for long term transdermal administration by forming a solution of a pharmaceutical and a freeze-dried latex polyacrylate copolymer in a solvent. U.S. Patent No. 5,906,830 discloses a method of manufacturing a supersaturated transdermal system comprising heating a mixture of undissolved drug and reservoir matrix material to a predetermined temperature, followed by cooling. These references, however, fail to disclose a method of making a stable transdermal device containing an active agent in amorphous form.

Finally, one problem encountered with drug delivery devices comprising supersaturated solutions is insufficient storage stability due to crystallization processes. Such crystallization processes result in a reduction in the amount of dissolved drug, and an increase in the amount of drug present in the crystalline state, thus reducing the efficacy of such a supersaturated device. To prevent crystallization processes in transdermal delivery devices and to be able to administer the therapeutically desired dose continuously, crystallization inhibitors are usually added to any delivery device. U.S. Patent Nos. 6,465,005, 5,676,968, 6,440,454, and 6,537,576 describe methods utilizing such crystallization inhibitors. However, the addition of non-adhesive crystallization inhibitors alters the adhesion properties of the adhesive by reducing its adhesiveness or by making the system softer. As such, the prior art fails to suggest a method of stabilizing an amorphous drug-in-adhesive matrix delivery device. Moreover, the prior art fails to suggest a method of reestablishing an amorphous drug-in-adhesive delivery device.

### SUMMARY OF THE INVENTION

In accordance with the present invention, a transdermal delivery device has been discovered comprising a backing layer, an adhesive matrix layer comprising a supersaturated concentration of at least one active agent substantially in amorphous form within an adhesive matrix, and a release liner. In accordance with another embodiment of the present invention, the active agent many be any active pharmaceutical ingredient capable of being provided in an amorphous form within a transdermal delivery device. In accordance with another embodiment of the present invention, the active agent is present in an amount of from about 0.1% to about 50% by weight of the adhesive matrix layer, preferably from about 1% to about 20% by weight of the adhesive matrix layer. In accordance with another embodiment of the present invention, the concentration of the active agent is from about 0.1% to about 1000% above the solubility of the active agent in the adhesive matrix.

In accordance with another embodiment of the present invention, the backing layer and the release liner are substantially non-crystallization inducing and free of crystallization nuclei or crystallization seeding particles. The backing layer is selected from the group consisting of polyester films, polyethelene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films. The release liner is selected from the group consisting of polyester liners, polyurethane liners, polyester liners with a silicone coating, polyurethane liners with a silicone coating, polyester liners with a fluorosilicone coating, polyurethane liners with a fluorosilicone coating, silicon coated polyester liners, silicon coated polyurethane liners, polyester liners with a fluoropolymer coating, and polyurethane liners with a fluoropolymer coating.

In accordance with another embodiment of the present invention, at least one of the backing layer and/or the release liner is larger than the adhesive matrix layer. In accordance with another embodiment of the present invention, at least one of the backing layer and/or the release liner is the same size as the adhesive matrix layer.

In accordance with another embodiment of the present invention, the adhesive matrix layer comprises an adhesive material selected from the group consisting of polyisobutylene, polysiloxane, acrylic adhesives, natural and synthetic rubber adhesives, and mixtures thereof. In accordance with another embodiment of the present invention, the adhesive material is present in an amount of from about 50% to about 99% by weight of the adhesive matrix, preferably in an amount of from about 60% to about 90% by weight of the adhesive matrix layer.

In accordance with another embodiment of the present invention, the adhesive matrix layer further comprises one or more tackifiers. The one or more tackifiers is selected from the group consisting of polybutenes, mineral oils, and polysiloxanes.

In accordance with another embodiment of the present invention, the adhesive matrix layer further comprises one or more cohesive enhancers. The one or more cohesive enhances is selected from the group consisting of colloidal silicone dioxide, zinc oxide, polyvinylpyrrolidine, acrylate copolymers, crosspovidone, bentonites, clays, and mixtures thereof.

In accordance with another embodiment of the present invention, the adhesive matrix layer further comprises one or more flux enhancers. The one or more flux enhancers is selected from the group consisting of propylene glycol, butylene glycol, dipropylene glycol, diethylene glycol, propyl palmitate, isopropyl palmitate, propyl myristate, glycerol monoesters, pendadecanol, pendadecalactone, octadecanol, oleyl alcohol, propylene glycol monoester, polyethylene glycol monoester, oleic acid.

In accordance with another embodiment of the present invention, the transdermal delivery device further comprises a drug release regulating membrane layer and a reservoir layer. In accordance with another embodiment of the present invention, at least one of the drug release regulating membrane layer and/or the reservoir layer contains one or more active agents.

In accordance with the present invention, a transdermal delivery device has been discovered comprising a backing layer, an adhesive matrix layer comprising a supersaturated concentration of oxybutynin substantially in amorphous form within an adhesive matrix, and a release liner. In accordance with another embodiment of the present invention, oxybutynin is present in an amount of from about 0.1% to about 50% by weight of the adhesive matrix, preferably the amount is from about 1% to about 20% by weight of the adhesive matrix. In accordance with another embodiment of the present invention, the concentration of oxybuytnin is from about 0.1% to about 10000% above the solubility of oxybutynin in the adhesive matrix, preferably the concentration of oxybutynin is from about 5% to about 5000% above the solubility of oxybutynin in the adhesive matrix, most preferably the concentration of oxybutynin is from about 10% to about 1000% above the solubility of oxybutynin in the adhesive matrix.

In accordance with another embodiment of the present invention, the backing layer and the release liner are substantially non-crystallization inducing and free of crystallization nuclei or crystallization seeding particles. The backing layer is selected from the group consisting of polyester films, polyethelene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films. The release liner is selected from the group consisting of polyester liners, polyurethane liners, polyester liners with a silicone coating, polyurethane liners with a silicone coating, polyester liners with a fluorosilicone coating, polyurethane liners with a fluorosilicone coating, silicon coated polyester liners, silicon coated polyurethane liners, polyester liners with a fluoropolymer coating, and polyurethane liners with a fluoropolymer coating.

In accordance with another embodiment of the present invention, at least one of the backing layer and the release liner is larger than the adhesive matrix layer.

In accordance with another embodiment of the present invention, the adhesive matrix comprises an adhesive material selected from the group consisting of polyisobutylene, polysiloxane, acrylic adhesives, natural and synthetic rubber adhesives, and mixtures thereof. In accordance with another embodiment of the present invention, the adhesive material is present in an amount of from about 50% to about 99% by weight of the adhesive matrix, preferably in an amount of from about 60% to about 90% by weight of the adhesive matrix layer.

In accordance with another embodiment of the present invention, the adhesive matrix layer further comprises one or more tackifiers.

In accordance with another embodiment of the present invention, the adhesive matrix layer further comprises one or more cohesive enhancers.

In accordance with another embodiment of the present invention, the adhesive matrix layer further comprises one or more flux enhancers.

In accordance with another embodiment of the present invention, the transdermal device further comprises a drug release regulating membrane layer and/or a reservoir layer.

In accordance with the present invention, a transdermal delivery device has been discovered comprising a backing layer, an adhesive matrix layer comprising a supersaturated concentration of at least one active agent substantially in amorphous form within the adhesive matrix, and a release liner, wherein the active agent is selected from the group consisting of piroxicam, fentanyl, naltrexone, scopolamine and a steroid. In accordance with another embodiment of the present invention, the steroid is selected from the group consisting of estrogens, progestogens, testosterone, noregestrel, norethindrone acetate, medroxyprogesterone acetate, levonorgestrel, and norelgestromin. In accordance with another embodiment of the present invention, the active agent is present in an amount of from about 0.1% to about 50% by weight of the adhesive matrix. In accordance with another embodiment of the present invention, the concentration of the active agent is from about 0.1% to about 10000% above the solubility of the active agent in the adhesive matrix.

In accordance with another embodiment of the present invention, the backing layer and the release liner are substantially non-crystallization inducing and free of crystallization nuclei or crystallization seeding particles. The backing layer is selected from the group consisting of polyester films, polyethelene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films. The release liner is selected from the group consisting of polyester liners, polyurethane liners, polyester liners with a silicone coating, polyurethane liners with a silicone coating, polyester liners with a fluorosilicone coating, polyurethane liners with a fluorosilicone coating, silicon coated polyester liners, silicon coated polyurethane liners, polyester liners with a fluoropolymer coating, and polyurethane liners with a fluoropolymer coating.

In accordance with another embodiment of the present invention, the at least one of the backing layer and the release liner is larger than the adhesive matrix layer.

In accordance with another embodiment of the present invention, the adhesive matrix comprises an adhesive material selected from the group consisting of polyisobutylene, polysiloxane, acrylic adhesives, natural and synthetic rubber adhesives, and mixtures thereof. In accordance with another embodiment of the present invention, the adhesive material is present in an amount of from about 50% to about 99% by weight of the adhesive matrix, preferably in an amount of from about 60% to about 90% by weight of the adhesive matrix layer.

In accordance with another embodiment of the present invention, the adhesive matrix layer further comprises one or more tackifiers.

In accordance with another embodiment of the present invention, the adhesive matrix layer further comprises one or more cohesive enhancers.

In accordance with another embodiment of the present invention, the adhesive matrix layer further comprises one or more flux enhancers.

In accordance with another embodiment of the present invention, the transdermal device further comprises a drug release regulating membrane layer and/or a reservoir layer. In accordance with the present invention, a method of preparing an adhesive matrix containing at least one active agent that is supersaturated and present in amorphous form has been discovered comprising the steps of: a) dissolving the active agent and an adhesive polymer in a solvent in an amount so as to provide the active agent at a subsaturated concentration in an adhesive matrix solution, b) casting the subsaturated active agent in the adhesive matrix solution to one of a release liner and a backing layer, c) removing the solvent at a temperature which is at, below, or above the melting point of the active agent to form a dry adhesive matrix in which the active agent is in a supersaturated concentration, and d) laminating the other of the release liner and the backing film to the supersaturated active agent in the dry adhesive matrix, so that the supersaturated active agent in the dry adhesive matrix is between the release liner and the backing layer. In accordance with another embodiment of the present invention, the active agent may be selected from any active pharmaceutical ingredient capable of being including in amorphous form within a transdermal delivery device, provided the active agent is not terazosin or rotigotine. In accordance with another embodiment of the present invention, the active agent is selected from the group consisting of oxybutynin, piroxicam, fentanyl, naltrexone, scopolamine, or a steroid.

In accordance with another embodiment of the present invention, the release liner and the backing layer are non-crystallization inducing and free of crystallization nuclei or crystallization seeding particles. In accordance with another embodiment of the present invention, the supersaturated active agent in the adhesive matrix further comprises one or more additives which are dissolved or undissolved but dispersed as liquid or solid particles in the dry adhesive matrix. In accordance with another embodiment of the present invention, the one or more additives are selected from the group consisting of penetration enhancers, crystal growth inhibitors, tackifiers, cohesive enhancers, plasticizers, and antioxidants. In accordance with another embodiment of the present invention, the one or more additives are present in an amount of from about 1% to about 50% by weight of the adhesive matrix. In accordance with another embodiment of the present invention, the one or more additives are present in an amount of from about 2% to about 25% by weight of the adhesive matrix. In accordance with another embodiment of the present invention, the solvent is present in an amount of from about 1% to about 200% more than the amount necessary to solubilize the active agent and the adhesive.

In accordance with another embodiment of the present invention, the solvent is selected from the group consisting of heptane, ethyl acetate, toluene, xylene, isopropanol, and ethanol.

In accordance with another embodiment of the present invention, the active agent is present in an amount of from about 0.1% to about 50% by weight of the adhesive matrix layer, preferably the active agent is present in an amount of from about 1% to about 20% by weight of the adhesive matrix layer.

In accordance with another embodiment of the present invention, the adhesive matrix material is present in an amount of from about 50% to about 99% by weight of the adhesive matrix layer, preferably the adhesive matrix material is present in an amount of from about 60% to about 90% by weight of the adhesive matrix layer.

In accordance with the present invention, a method of preparing an adhesive matrix containing at least one active agent that is supersaturated and present in amorphous form has been discovered comprising the steps of: a) admixing the active agent with an adhesive matrix at a supersaturated concentration, b) heating the supersaturated concentration of the active agent in the adhesive matrix to a temperature which allows the active agent to be completely dissolved and uniformly dispersed in the adhesive matrix to create a hot melt, c) casting the hot melt to one of a release liner and a backing layer, at a predetermined temperature, and d) laminating the other of the release liner and the backing layer to the hot melt, so that the hot melt is between the release liner and the backing layer. In accordance with another embodiment of the present invention, the active agent may be selected from any active pharmaceutical ingredient capable of being including in amorphous form within a transdermal delivery device, provided the active agent is not terazosin or rotigotine. In accordance with another embodiment of the present invention, the active agent is selected from the group consisting of oxybutynin, piroxicam, fentanyl, naltrexone, scopolamine, or a steroid.

In accordance with another embodiment of the present invention, the release liner and the backing layer are non-crystallization inducing and free of crystallization nuclei or crystallization seeding particles. In accordance with another embodiment of the present invention, the hot melt further comprises one or more additives which are dissolved or undissolved but dispersed in the adhesive matrix. In accordance with another embodiment of the present invention, the one or more additives are selected from the group consisting of penetration enhancers, crystal growth inhibitors, tackifiers, cohesive enhancers, plasticizers, and antioxidants. In accordance with another embodiment of the present invention, the one or more additives are present in an amount of from about 1% to about 50% by weight of the adhesive matrix.

In accordance with another embodiment of the present invention, the one or more additives are present in an amount of from about 2% to about 25% by weight of the adhesive matrix. In accordance with another embodiment of the present invention, the active agent is present in an amount of from about 0.1% to about 50% by weight of the adhesive matrix, preferably the active agent is present in an amount of from about 1% to about 20% by weight of the adhesive matrix.

In accordance with another embodiment of the present invention, the adhesive matrix is present in an amount of from about 50% to about 99% by weight of the adhesive matrix layer, preferably the adhesive matrix is present in an amount of from about 60% to about 90% by weight of the adhesive matrix layer.

In accordance with another embodiment of the present invention, is a method of reestablishing the favored internal adhesive matrix environment of a transdermal drug delivery device having a backing layer, an adhesive matrix layer having a supersaturated concentration of an active agent substantially in the amorphous form within the adhesive matrix layer, and a release liner, has been discovered comprising curing the transdermal delivery device. In accordance with another embodiment of the present invention, the active agent may be selected from any active pharmaceutical ingredient capable of being including in amorphous form within a transdermal delivery device. In accordance with another embodiment of the present invention, the active agent is selected from the group consisting of oxybutynin, piroxicam, fentanyl, naltrexone, scopolamine, or a steroid.

In accordance with another embodiment of the present invention, the heat curing comprises heating the transdermal delivery device to a temperature at which the drug completely dissolves or to a temperature about 20°C above the melting point of the active agent.

In accordance with another embodiment of the present invention, the curing comprises subjecting the device to oven infrared beams. In accordance with another embodiment of the present invention, the curing is performed for a duration ranging from about 1 second to about 10 minutes, preferably ranging from about 3 seconds to about 5 minutes, most preferably ranging from about 5 seconds to about 60 seconds.

In accordance with yet another embodiment of the present invention, is a method of storing and protecting a transdermal delivery device having a backing layer, an adhesive matrix layer comprising a supersaturated concentration of at least one active agent substantially in amorphous form within the adhesive matrix, and a release liner wherein the method comprises packaging the transdermal delivery device in a pouch. The pouch may be the same size or larger than the release liner. The pouch may be comprised of paper, polymer film(s), metal foil(s), or any combination thereof.

The stability of the amorphous form of an active agent at a storage temperature is dependent on the active agent's glass transition temperature (Tg) and the difference between the glass transition temperature and the storage temperature. Applicants have found that an amorphous form of an active agent having a higher Tg is more stable than an amorphous form of an active agent having a lower T_{g}.

Specifically, Applicants have found that the Tg of the amorphous forms of oxybutynin, fentanyl, and scopolamine are very low (lower than the normal storage temperature of 20°C to 25°C). For example, Applicants have found that the glass transition temperature of the amorphous form of oxybutynin is about -20°C, which is about 40°C to about 45°C lower than room temperature. On the other hand, the glass transition temperature of terazosin and rotigotine are higher than the normal storage temperature. As such, amorphous forms of oxybutynin, fentanyl, and scopolamine are more difficult to stabilize than the amorphous forms of terazosin or rotigotine.

Moreover, Applicants have found that an amorphous-drug-in-adhesive provides a higher skin flux relative to transdermal delivery devices containing crystalline forms of an active agent or active agents in a subsaturated solution. Further, Applicants have discovered a method of forming transdermal delivery devices incorporating amorphous forms of active agents which are typically very difficult to stabilize in the amorphous form in transdermal delivery devices.

### DETAILED DESCRIPTION

One embodiment of the present invention is a transdermal delivery device comprising a backing layer, an adhesive matrix layer comprising a supersaturated concentration of at least one active agent substantially in amorphous form within an adhesive matrix, and a release liner.

As used herein, "transdermal" means delivery of a drug by passage into and through the skin or mucosal tissue. Hence the terms "transdermal" and "transmucosal" are used interchangeably unless specifically stated otherwise. Likewise the terms "skin," "derma," "epidermis", "mucosa," and the like shall also be used interchangeably unless specifically stated otherwise.

The backing layer is a flexible substrate which provides a barrier to active drug migration away from the intended direction of drug delivery. Any well-known backing layer which satisfies this purpose can be used in the present invention.

Preferably, the backing layer is composed of materials that are substantially non-crystallization promoting and free of crystallization nuclei. Such backing layers aid in the preservation of the amorphous drug-in-adhesive matrix by preventing crystal formation. Examples of materials from which the backing layer may be composed include polyethylene terephthalate, various nylons, polypropylenes, polyesters, polyester/ethylene-vinyl acetates, metalized polyester films, polyvinylidene chloride, metal films such as aluminum foils, polyvinylidene fluoride films, or mixtures or copolymers thereof.

Other backing layers include ethylene vinyl acetate films laminated to a polyester, ethylene vinyl acetate films laminated to a metalized polyester, Mediflex® 1200 available from Mylan Technologies, Inc., Mediflex® 1501 from Mylan Technologies Inc., Mediflex® 1201 available from Mylan Technologies, Inc., Mediflex® 1502 available from Mylan Technologies, Inc., Dupont polyester type S available from Dupont, Dow BLF® 2050 available from The Dow Chemical Company, 3M™ Scotchpak® 1109 available from 3M, 3M™ Scotchpak® 9723 available from 3M, 3M™ Scotchpak® 9733 available from 3M, 3M™ Scotchpak® 9735 available from 3M and 3M™ Scotchpak® 9730 available from 3M.

Silicone coated polyethylene backings, such as Mediflex® 1000 coated with a silicone layer, 3M™ Cotran® 9722 coated with a silicone layer, and 3M™ Cotran™ 9720 coated with a silicone layer, preserve the amorphous form of the drug in the adhesive matrix. Similarly, silicone coated polyester backings, such as Mediflex® 1200 coated with a silicone layer, also preserves the amorphous form of drug in adhesive.

In some embodiments, the backing layer may be the same size as the adhesive matrix layer and/or may be the same size as the release liner. In other embodiments, the backing layer may be oversized as compared with the adhesive layer, i.e. the backing layer may be larger than the adhesive layer. In yet other embodiments, the backing layer may range from about 0.01mm to at least 10mm larger than the adhesive matrix layer, preferably ranging from about 0.05mm to about 5mm larger than the adhesive matrix layer, and most preferably ranging from about 0.1mm to about 3mm larger than the adhesive matrix layer. Use of an oversized backing layer helps prevent the adhesive matrix from becoming distorted or relaxing during the handling and shipping processes. Such an oversized backing layer may help prevent crystal growth, especially when the devices are stored for long periods of time or when they are exposed to temperature fluctuations.

Adjacent to the backing layer is an adhesive matrix layer comprising a supersaturated concentration of at least one active agent dissolved and/or dispersed in an adhesive material.

The "adhesive material" or "adhesive matrix" (used interchangeable) may be any biocompatible polymer or polymeric material known in the art. The adhesive matrix material may be selected from silicones, natural and synthetic rubbers, polyisobutylene ("PIB"), neoprenes, polybutadienes, polyisoprenes, polysiloxanes, acrylic adhesives including cross-linked and uncross-linked acrylic copolymers, vinyl acetate adhesives, polyacrylates, ethylenevinylacetate copolymers, styrene-isoprene copolymers, polyurethanes, plasticized weight polyether block amide copolymers, plasticized styrene-rubber block copolymers, and mixtures thereof.

The adhesive matrix material may also be selected from acrylic adhesives and polyacrylate adhesives sold under the trademark Duro-Tak 80-1194, 80-1196, 80-1197, 2287, 2516 2852, 387-2051, 387-2052, 387-2054, 387-2287, 387-2353, 387-2510, 387-2516, 387-2620, 387-2825, 387-2070, 87-2074, 87-2097, 87-2100, 87-2154, 87-2194, 87-2196, 87-2852 and 87-2979 by National Starch and Chemical Corporation, Bridgewater, N.J., USA. Other suitable acrylic adhesives include those sold under the trademark Gelva--Multipolymer Solution GMS 737, 788, 263, 1151, 1159, 1430, 1753, 2450, 2465, 2480, 2495, 2497 and 2539 by Monsanto, St Louis, Mo. USA.

Pressure sensitive silicone containing adhesives are available from Dow Corning under the trademark BIO-PSA® 7-4101, 7-4201, 7-4301, 7-4102, 7-4202, 7-4302, 7-4103, 7-4203, and 7-4303 and may be utilized as an adhesive matrix material.

The adhesive matrix material is generally present in the adhesive matrix layer in an amount ranging from about 50% to about 99% by weight of the adhesive matrix layer, preferably ranging from about 60% to about 90% by weight of the adhesive matrix layer.

The active agent is dissolved or dispersed within the adhesive matrix and present substantially in amorphous form. As used herein, the terms "active agent" or "drug" (used interchangeably) are used to describe the principal active pharmaceutical ingredient of the transdermal delivery device, which is a biologically active compound or mixture of compounds that has a therapeutic, prophylactic and/or physiological effect on the wearer of the device. As used herein, the term "substantially" means to meet the criteria in such measure that one skilled in the art would understand that the benefit to be achieved, or the condition or property value desired, is met.

The active agent may be any active pharmaceutical ingredient capable of being provided in an amorphous form within a transdermal delivery device, provided the active agent is not terazosin or rotigotine.

Non-limiting examples of active agents include antiinflammatory substances, opioid receptor antagonists, anticholinergics, coronary dilators, cerebal dilators, peripheral vasodilators, alpha-adrenergic blockers, antiinfectives, psychotropics, anti-manics, stimulants, antihistamines, decongestants, gastro-intestinal sedatives, anti-anginal drugs, vasodilators, anti-arrhythmics, antihypertensive drugs, vasoconstrictors, migraine treatments, anti-coagulants and anti-thrombotic drugs, analgesics, antipyretics, hypnotics, sedatives, anti-emetics, anti-nauseants, anti-convulsants, neuromuscular drugs, hyper- and hypoglycemic agents, thyroid and anti-thyroid preparations, diuretics, anti-spasmodics, anti-emetic, uterine relaxants, anti-obesity drugs, anabolic drugs, erythropoietic drugs, anti-asthmatics, bronchodilators, expectorants, mucolytics, anti-uricemic drugs, narcotics, anti-depressants, agents for treating alcohol abuse or dependence and the like.

In some embodiments of the present invention, the active agent is oxybutynin. As used herein, the term "oxybutynin" is used to designate oxybutynin, the salts, solvates, and hydrates of oxybutynin, and the related compounds thereof. In one preferred embodiment, the active agent is oxybutynin in the form of a free base.

In other embodiments of the present invention, the active agent is scopolamine. As used herein, the term "scopolamine" is used to designate scopolamine, the salts, solvates, and hydrates of scopolamine, and derivative compounds thereof (including, but not limited to, butylscopolamine).

In yet other embodiments of the present invention, the active agent is naltrexone. As used herein, the term "naltrexone" is used to designate naltrexone, the salts, solvates, and hydrates of naltrexone, and the related compounds thereof.

In yet other embodiments of the present invention, the active agent is a steroid. Examples of steroids useful herein include progestogens such as allylestrenol, anagestone, chlomardinone acetate, delmadinone acetate, demegestone, desogestrel, 3-keto desogestrel, dimethisterone, drospirenone, dydrogesterone, ethisterone, ethynodiol, flurogestone acetate, gestodene, gestonorone caproate, 17-hydroxy-16-methylene-.delta.-progesterone, 17.alpha. hydroxyprogesterone, hydroxyprogesterone, hydroxyprogesterone acetate, hydroxyprogesterone caproate, levonorgestrel, lynestrenol, medrogestone, medroxyprogesterone, medroxyprogesterone acetate, megestrol acetate, melengestrol, norethindrone, norethindrone acetate, norethynodrel, norgesterone, norgestimate, norgestrel, norgestrienone, norethisterone, norethynodrel, norvinisterone, pentagestrone, progesterone, promegestone, trengestone.

Other examples of steroids include: estrogens such as nonsteroidal estrogens such as benzestrol, broparoestrol, chlorotrianisene, dienestrol, diethylstilboestrol, diethylstilboestrol dipropionate, dimestrol, fosfestrol, hexoestrol, methallenestril and methestrol, and steroidal estrogens as colpormon, conjugated estrogenic hormones, equilenin, equilin, estradiol and its esters (e.g., estradiol benzoate, valerate, cyprionate, decanoate and acetate), estriol, estrone, ethinyl estradiol, estradiol benzoate, mestranol, moxestrol, mytatrienediol, quinestradiol, quinestrol.

Yet other examples of steroids include corticosteroids such as betamethasone, betamethasone acetate, cortisone, hydrocortisone, hydrocortisone acetate, corticosterone, fluocinolone acetonide, prednisolone, prednisone and triamcinolone; and androgens and anabolic agents such as aldosterone, androsterone, testosterone and methyl testosterone.

Androgens such as boldenone, cloxotestosterone, fluoxymesterone, mestanolone, mesteronolone, 17-methyltestosterone, 17.alpha.-methyltestosterone 3-cyclopentyl enol ether, norethandrolone, normethandrone, oxandrolone, oxymesterone, oxymetholone, prasterone, stanolone, stanolozol, testosterone, tiomesterone.

Glucocorticoids such as 21-acetoxypregnenolone, alclometasone, algestone, amcinonide, beclomethasone, bethamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, flucinolone acetonide, fluocinonide, fluocortin butyl, fluocortolone, fluorometholone, fluperolone acetate, flupredninene acetate, fluprednisolone, flurandrenolide, fluticasone propionare, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylaminoacetate, prednisolone sodium phosphate, prednisone, prednival, prednylidene, rimexolone, tixocortol, triamcinolone, triamcinolone acetonide, triamcinolone benetonide, triamcinolone hexacetonide.

Additional steroids include noregestrel, levonoregestrel, norelgestromin, and derivatives thereof.

In preferred steroid embodiments, the steroids are selected from estradiol, norelgestramine, and testosterone.

In yet other embodiments of the present invention, the active agent is piroxicam. As used herein, the term "piroxicam" is used to designate piroxicam, the salts and hydrates of piroxicam, and the related compounds thereof.

In yet other embodiments of the present invention, the active agent is fentanyl. As used herein, the term "fentanyl" is used to designate fentanyl, the salts and hydrates of fentanyl, and the related compounds thereof.

The active agent is generally present in an amount ranging from about 0.1% to about 50% by weight of the adhesive matrix layer, preferably from about 1% to about 20% by weight of the adhesive matrix layer.

The active agent is present in a supersaturated concentration within the adhesive matrix. In one embodiment, the active agent concentration ranges from about 0.1% to 10000% above the solubility of the active agent in the adhesive matrix. In another embodiment, the active agent concentration ranges from about 5% to about 5000% above the solubility of the active agent in the adhesive matrix. In yet another embodiment, the concentration of active agent ranges from about 10% to about 1000% above the solubility of the active agent in the adhesive matrix.

The amount of active agent present in amorphous form within the device is generally in an amount ranging from about 1% to about 100% by weight of the total amount of active agent, preferably ranging from about 20% to about 80% by weight of the total amount of active agent, and most preferably ranging from about 40% to about 60% by weight of the total amount of active agent.

The adhesive matrix layer may contain one or more additives selected from tackifiers, cohesive enhancers, permeation enhancers, crystal growth inhibitors, plasticizers, antioxidants, flux enhancers, penetration enhancers, and/or other pharmaceutically acceptable additives or excipients. The additives are generally present in the composition in an amount ranging from about 1% to about 50% by weight of the adhesive matrix layer, and preferably ranging from about 2% to about 25% by weight of the adhesive matrix layer.

In some embodiments, the adhesive matrix layer contains one or more tackifiers. As used herein, the term "tackifier" refers to materials other than PIB that are added to adhesives to increase their tack or stickiness. If tackifiers are included, they are generally present in an amount ranging from about 1% to about 50% by weight of the adhesive matrix layer, preferably from about 5% to about 40% by weight of the adhesive matrix layer. Tackifiers are generally comprised of materials such as naturally occurring resinous, rosinous materials, or truly synthetic polymer materials. Examples of tackifiers include hydrogenated or partially hydrogenated glycerol esters of rosin, polyterpenes, polybutenes, or polysiloxanes.

In some embodiments, the adhesive matrix layer contains one or more cohesive enhancers. The addition of a cohesive enhancer into the adhesive matrix increases the adhesive matrix's storage modulus. Cohesive enhancers are generally present in an amount ranging from about 0.1% to about 25% by weight of the adhesive matrix layer, preferably from about 1% to about 15% by weight of the adhesive matrix layer. Examples of cohesive enhancers include colloidal silicone dioxide, zinc oxide, clays, bentonite, polyvinylpyrrolidone ("PVP"), polyvinylpyrrolidone-co-vinylacetate, Eudragit® copolymers, ethyl cellulose or crosspovidone.

In some embodiments, the adhesive matrix layer contains one or more flux enhancers as part of the drug formulation. As used herein, the term "flux enhancer" is used to describe a compound which aids in increasing the permeability of a drug through the skin to the blood stream. If flux enhancers are included, they are generally present in an amount ranging from about 0.1% to about 40% by weight of the adhesive matrix layer, preferably from about 1% to about 20% by weight of the adhesive matrix layer.

Suitable flux enhancers include dimethylsulfoxide (DMSO), dimethyl formamide (DMF), N,N-dimethylacetamide (DMA), decylmethylsulfoxide (C.sub.10 MSO), polyethylene glycol monolaurate (PEGML), propylene glycol (PG), propylene glycol monolaurate (PGML), butylene glycol, dipropylene glycol, diethylene glycol, propyl palmitate, isopropyl palmitate, propyl myristate, glycerol monoesters, glycerol monolaurate (GML), propylene glycol monoester, polyethylene glycol monoester, methyl laurate (ML), lauryl lactate (LL), isopropyl myristate (IPM), terpenes such as menthone, C₂-C₆ diols, particularly 1,2-butanediol, lecithin, the 1-substituted azacycloheptan-2-ones, 1-n-dodecylcyclazacycloheptan-2-one, C2 to C18 alcohols, triacetin, and the like. Vegetable oil permeation enhancers, as described in U.S. Pat. No. 5,229,130, may also be used. Such oils include safflower oil, cotton seed oil and corn oil.

Adjacent to the adhesive matrix layer is a release liner. Release liners well known in the art can be used in the present invention. Examples of materials from which the release liner may be composed include polyethylene terephthalate/silicone (i.e. polydimethyl siloxane) ("PET/SI"), polyethylene terephthalate/aluminized polyester coated with silicone (i.e. polydimethyl siloxane) ("PET/MET/SI"), polyester or polyurethane liners with a silicone coating, polyester or polyurethane liners with a fluorosilicone coating, or polyester or polyurethane liners with a silicon coating.

Preferably, the release liner is composed of materials that are substantially non-crystallization promoting and free of crystallization nuclei. Such release liners aid in the preservation of the amorphous drug-in-adhesive matrix. Specific release liners include Medirelease® 2249, Medirelease® 2226, Medirelease® 2500, 3M™ Scotchpak® 1020, 3M™ Scotchpack® 1022, 3M™Scotchpak® 9741, 3M™ Scotchpak® 9742, 3M™ Scotchpak® 9744, CPFilms Inc. Clearsil® UV5A and CPFilms Inc., Clearsil® UV510, CPFilms Inc. Sil® UV5A and CPFilms Inc. Sil® UV510.

In some embodiments, the release liner may be the same size as the adhesive matrix layer and/or may be the same size as the backing layer. In other embodiments, the release liner may be larger than the adhesive matrix layer and/or may be larger than the backing layer. In yet other embodiments, the release liner may range from about 0.1mm to at least about 20mm larger than the diameter of a round backing layer or a round adhesive matrix layer, preferably ranging from about 0.5mm to about 10mm larger than the backing layer or adhesive matrix layer, and most preferably ranging from about 1mm to about 5mm larger than the backing layer or adhesive matrix layer. The release liner may also range from about 0.1mm to at least about 20mm larger than each side of a rectangular or square backing layer or adhesive matrix layer, preferably ranging from about 0.5mm to about 10mm larger than the backing layer or adhesive matrix layer, and most preferably ranging from about 1mm to about 5mm larger than the backing layer or adhesive matrix layer.

Use of an oversized release liner helps prevent the adhesive matrix from becoming distorted or relaxing during the handling and shipping processes. Such an oversized release liner may help prevent crystal growth, especially when the transdermal delivery devices are stored for long periods of time, are exposed to temperature fluctuations, or are exposed to shipping and/or moving stresses. For example, when an adhesive matrix is laminated between a backing layer and a release liner that is the same size as the adhesive matrix, coupled with heat curing, crystal growth is observed to start from the edge of the patch and progress toward the center. However, when that same adhesive matrix is laminated between a backing layer and an oversized release liner, coupled with heat curing, there is no observed crystal growth even after the patch is stored for two months, subjected to ten cycles of freeze and thaw stability testing, or subjected to repeated microscopic observations.

In one embodiment, the adhesive matrix layer is laminated between an oversized release liner and an oversized backing layer. In another embodiment, the adhesive matrix layer is laminated between an oversized release liner and backing layer of the same size as the adhesive layer. In yet another embodiment, the adhesive matrix layer is laminated between an oversized release liner and a backing layer of the same size as the adhesive layer with an overlay film above the backing layer. If an overlay film is utilized, the overlay may be the same material or may be a different material than the release liner.

The overlay is typically the same size as the oversized release liner, but larger in size than the backing layer. The overlay layer may be about 0.01mm to at least about 20mm larger than the diameter of a round backing layer or than each dimension of a rectangular or square backing layer. Moreover, the overlay typically covers the edge of the backing layer. Examples of overlay films include 3M™ Scotchpak™1022, Medirelease®2249, and Medirelease® 2226.

The transdermal delivery device may include one or more additional layers. One such additional layer is a reservoir layer. Preferably, the reservoir layer is composed of materials that are free of crystallization seeding particles. The reservoir layer may contain one or more active agents and one or more pharmaceutically acceptable additives.

In general, the reservoir layer is a layer that is placed between a backing film and a drug release regulating membrane layer. In such an example, the reservoir layer contains an amount of active agent which is higher than an amount of active agent present in an adhesive matrix layer (which is located between the membrane layer and the release liner). The active agent(s) may be in amorphous form in an adhesive matrix or in a gel in the reservoir layer. The skin contact layer may include no active agent or may include at least one active agent substantially in amorphous form.

The transdermal delivery system may also include a drug release regulating membrane layer. Such a membrane layer may be present in a drug delivery device beneath, and typically immediately adjacent to, the drug reservoir layer, and generally between the drug reservoir itself and an adhesive matrix layer which affixes the device to the skin.

Representative materials useful for forming rate-controlling membrane layers include polyolefins such as polyethylene and polypropylene, polyamides, polyesters, ethylene-ethacrylate copolymers, ethylene-vinyl acetate copolymers, ethylene-vinyl methylacetate copolymers, ethylene-vinyl ethylacetate copolymers, ethylene-vinyl propylacetate copolymers, polyisoprene, polyacrylonitrile, ethylenepropylene copolymers, ethylene-vinyl acetate copolymer, and the like. Preferably, the drug release regulating membrane layer is composed of materials that are non-crystallization promoting and free of crystallization nuclei.

The drug release regulating membrane layer may contain one or more active agents and one or more pharmaceutically acceptable additives.

The transdermal delivery device unit dosage form may be placed in appropriate packaging for storage and protection, such as paper, polymer films, and/or metal foil pouches, until they are to be applied in transdermal treatment. The packaging or pouch may be the same size or larger than the overlay or release liner in one or all of the dimensions. The packing or pouch may range from about 0.1mm to about 20mm larger than the overlay and/or release liner, preferably ranging from about 0.2mm to 10mm larger than the overlay and/or release liner, most preferably ranging from about 0.5mm to about 2mm larger than the overlay and/or release liner. A tight fit between the patch and pouch prevents movement of the patch inside the pouch and thus prevents the adhesive edge of the patch from being damaged during shipping and handling processes.

Two methods of preparing an adhesive matrix containing at least one active agent that is supersaturated and present in an amorphous form are provided. A first method comprises the following steps: first, the active agent and an adhesive polymer are dissolved in a solvent system so as to provide the active agent in an adhesive matrix solution at a subsaturated concentration (but once the solvent is removed, the active agent will be at a supersaturated concentration in the dry adhesive matrix); second, the subsaturated active agent in the adhesive matrix solution is cast to at least one of a release liner or a backing layer; third, the solvent is removed from the adhesive matrix solution at a temperature which is at, below, or above the melting point of the active agent to spontaneously form the supersaturated concentration of amorphous drug-in-adhesive matrix; and fourth, the other of a release liner or a backing film is laminated to the supersaturated active agent in the adhesive matrix, so that the supersaturated active agent in the adhesive matrix is between the release liner and the backing layer. The active agent may be any active pharmaceutical ingredient capable of being including in an amorphous form within a transdermal delivery device, provided the active agent is not terazosin or rotigotine. In preferred embodiments, the drug is selected from oxybutynin, piroxicam, fentanyl, naltrexone, scopolamine, or a steroid.

In one embodiment of this first method, the release liner and/or the backing layers are non-crystallization promoting and free of crystallization nuclei. In another embodiment of this first method, the supersaturated drug-in-adhesive-matrix contains one or more additives or excipients which are dissolved or undissolved but dispersed as liquid or solid particles in the adhesive matrix. The amount of solvent necessary for this method ranges from about 1% to about 200% more than the amount of solvent necessary to solubilize the drug and adhesive. The solvent may be chosen from organic solvents including pentanes, hexanes, heptanes, octanes, ethyl acetate, ethanol, isopropanol, toluene, xylenes and mixtures thereof. For the adhesive system, if the type of solvent present in the adhesive matrix solution has a lower solubility for the drug than for the adhesive, a second solvent may be added to dissolve both the drug and the adhesive. The ratio of the first solvent to the second solvent is the ratio at which both the adhesive and the drug can be completely dissolved to form a single phase. An optimum ratio and the amount of each of the two solvents required to form a single phase solution of the adhesive and drug varies from drug to drug and varies with the amount of the drug utilized.

A second method of preparing an adhesive matrix containing at least one active agent that is supersaturated and present in amorphous form comprises the following steps: admixing the active agent with an adhesive matrix at a supersaturated concentration; heating the adhesive matrix to a temperature which allows the active agent to be completely dissolved in the adhesive melt, or melted and finely dispersed in the adhesive matrix, to create a hot melt; casting the hot melt to at least one of a release liner or a backing layer; and laminating the other of a release liner or a backing layer to the hot melt, so that the hot melt is between the release liner and the backing layer. As the hot melt is cooled to ambient temperature, the amorphous drug-in-adhesive matrix is spontaneously formed, whereby the solid amorphous drug is finely dispersed in the adhesive matrix. The active agent may be any active pharmaceutical ingredient capable of being including in an amorphous form within a transdermal delivery device. In preferred embodiments, the drug is selected from oxybutynin, piroxicam, fentanyl, naltrexone, scopolamine, or a steroid.

In one embodiment of this first method, the hot melt contains one or more additives or excipients which are dissolved or undissolved but dispersed in the adhesive matrix.

In another embodiment of this method, the release liner and the backing layer are non-crystallization promoting and free of crystallization nuclei.

Crystalline forms of drugs are the most thermodynamically stable forms. As a result, drug molecules will self-organize themselves in such a structurally ordered way as to form crystals with the lowest possible amount of energy. Under thermodynamically favored conditions, amorphous forms of drugs or less favored crystal forms will eventually convert to the most stable crystal form. One way in which crystallization or conversion may occur is through the presence of pre-existing drug crystals or other solid particles (nuclei) present in the adhesive matrix which provide support for crystal growth formation. This process is termed crystal seeding. Thus, to avoid crystal growth formation, a backing layer and/or a release liner that are non-crystallization promoting and free of crystallization nuclei is utilized. Such a non-crystallization promoting backing layer and/or a non-crystallization release liner has been shown to prevent crystal formation and growth in an amorphous drug-in-adhesive-matrix. Moreover, utilization of an oversized backing layer or an oversized release liner in a patch may further avoid crystallization of the amorphous form. Indeed, use of such an oversized release liner or oversized backing layer helps prevent the edge of the adhesive matrix from becoming distorted or relaxing during the handling and shipping processes or when the devices are stored for long periods of time or are exposed to temperature fluctuations.

A solid drug can exist in one or more crystalline forms and in amorphous form. Structurally ordered molecules form crystals. Of all the possible crystalline forms, one crystalline form is most thermodynamically stable among the crystalline forms. The amorphous form of a drug, however, is meta stable, meaning it is thermodynamically unstable. Unlike crystalline forms, amorphous drug molecules are structurally organized in a random order. Under thermodynamically favored conditions, the less stable crystalline forms and amorphous form will eventually convert to the most stable crystalline form. Precisely how long a drug retains the meta stable amorphous form before crystallization initiates is dependent on the internal and external environments. Favored external environment conditions include storing an amorphous drug product at a low temperature, e.g., storing the amorphous form of the drug at a temperature that is not more than 50°C higher than its Tg, and not disturbing the matrix containing the amorphous drug. Favored internal environments that can extend the life of the amorphous form include those adhesive matrix types that can reduce the movement of amorphous drug molecules by forming hydrophobic associations and/or hydrogen bonds between the matrix molecules and drug molecules.

It is desirable to be able to melt or redissolve the crystallization nuclei and reestablish the internal adhesive matrix for a drug in amorphous form should crystallization initiate. As such, a method of reestablishing the internal adhesive matrix environment for the amorphous form comprises heat curing a die-cut patch at a particular temperature for a sufficient period of time. Preferably, the heat curing is done at the temperature of the melting point of the active agent up to a temperature about 20°C above the melting point of the active agent. Preferably, the heat curing is done either after die-cutting and after packaging or after die-cutting and before packaging. Preferably, heat curing is performed before any crystals are formed or before a substantial amount of crystals are formed. Heat curing sources include oven electric heating and infra-red beams.

The following examples further illustrate the invention and its unique characteristics. These examples are not intended to limit the invention in any manner.

In examples 1 through 9, the active agent is oxybutynin in the form of a free base. Its solubility in water at pH 7.4 is about 15 µg/ml. In these examples, the solubility of oxybutynin base in dry adhesive Bio-PSA 7-4302 and in a mixture of dry adhesive Bio-PSA 7-4302 and colloidal silicon dioxide (CSD) is less than 3% by weight. If the adhesive matrix contains 2.5% isopropyl palmitate as a penetration enhancer, the solubility of oxybutynin base remains about 3%.

**Table 1. Compositions of polysiloxane based dry adhesive matrices**

| | Weight Percent | | | | | | | | |
|---|---|---|---|---|---|---|---|---|---|
| Example # | 1 | 2 | 3 | 4 | 5 | 6 | 7 | 8 | 9 |
| Oxybutynin base | 24.85 | 17.5 | 15 | 17.5 | 17.5 | 10 | 10 | 10 | 10 |
| Bio-PSA 7-4302 | 69.65 | 77 | 0 | 77 | 79.5 | 84.5 | 87 | 86 | 85 |
| Bio-PSA-4301 | 0 | 0 | 82.5 | 0 | 0 | 0 | 0 | 0 | 0 |
| IPP | 2.5 | 2.5 | 2.5 | 2.5 | 0 | 2.5 | 0 | 0 | 0 |
| CSD | 3 | 3 | 0 | 3 | 3 | 3 | 3 | 4 | 5 |
| Total | 100 | 100 | 100 | 100 | 100 | 100 | 100 | 100 | 100 |

| | | | | | | | | | |
|---|---|---|---|---|---|---|---|---|---|
| IPP: isopropyl palmitate. CSD: colloidal silicon dioxide. | | | | | | | | | |

Example 1. Preparation of crystalline drug-in-polysiloxane adhesive matrix with a solvent method

In this example, 42.84 grams of Bio-PSA 7-4302 (60% polysiloxane adhesive solid dissolved in ethyl acetate), 7.00 grams of micronized oxybutynin base, 1.00 grams of isopropyl palmitate and 1.20 grams of colloidal silicon dioxide (CSD) were added to a glass jar. After the contents were sonicated for 1 minute, the solid was admixed with a wood spatula. The content was further sonicated for 9 minutes to solvate the CSD and stirred with a mechanical mixer for 3 minutes. After the glass jar was rolled overnight to remove air, a liquid blend, containing dispersed CSD and some undissolved oxybutynin base crystals, was obtained. The blend was coated to a fluropolymer coated release liner Scotchpak™ 1022, dried at room temperature for 5 min. and at 50°C for 90 minutes. A polyester backing film (Mediflex 1200, smooth polyester side) was laminated to the dry adhesive. The laminate was opaque right after it was made and became clear, containing dense fine clear crystals. The crystals were observed both visually and microscopically. A three-layer patch was made by die-cutting the laminate. The drug-in-adhesive matrix was sandwiched between a backing film and a release liner. The composition of the dry adhesive matrix is described in Table 1. Because the drug concentration in the adhesive matrix was higher than its solubility (about 3% by weight), the drug was saturated in the adhesive matrix. Thus, the adhesive matrix contained both dissolved oxybutynin base and undissolved oxybutynin base. In this example, the undissolved oxybutynin base was present in crystalline form dispersed in the adhesive matrix. As such, the patch was a crystalline drug-in-adhesive matrix patch. In example 1, the oxybutynin base crystals were not completely dissolved in the blend. Therefore, the blend contained dissolved oxybutynin base and undissolved oxybutynin base crystals, i.e. the blend was a saturated solution of oxybutynin base. This was done by controlling the ratio of oxybutynin base to ethyl acetate. The undissolved oxybutynin base crystals, which were crystallization nuclei, promoted fast recrystallization of dissolved oxybutynin base after the solvent was removed. Because the drying temperature (50°C) was lower than the melting point of oxybutynin base crystals, crystals carried over from the blend and crystals formed during the drying process were not melted.

Example 2. Preparation of crystalline drug-in-polysiloxane adhesive matrix with a solvent method

The composition of the adhesive matrix in this example is described in Table 1. The laminate and patch of example 2 were prepared similarly to example 1. The patch of example 2 is a crystalline drug-in-adhesive matrix patch.

Example 3. Preparation of crystalline drug-in-polysiloxane adhesive matrix with a solvent method

The adhesive matrix for this example was prepared similarly to example 1, but Bio-PSA 7-4302 was replaced with Bio-PSA 7-4301. Bio-PSA 7-4301 is a solution of a polysiloxane adhesive in heptane. The polysiloxane in Bio-PSA-7-4301 is exactly the same as the polysiloxane adhesive in Bio-PSA7-4302. Because heptane is a poor solvent for oxybutynin base, most of the oxybutynin base crystals were not dissolved in the blend. The undissolved oxybutynin base crystals seeded fast recrystallization of pre-dissolved oxybutynin base. The patch obtained was a crystalline drug-in-adhesive matrix laminate.

Example 4. Preparation of amorphous drug-in-polysiloxane adhesive matrix method with a solvent method

In this example, 89.53 grams of Bio-PSA 7-4302 (60.2% polysiloxane adhesive solid dissolved in ethyl acetate), 12.25 grams of micronized oxybutynin base, 1.75 grams of isopropyl palmitate, 2.10 grams of colloidal silicon dioxide (CSD) and 10.73 grams of additional ethyl acetate were added to a glass jar. After the contents were sonicated for 1 minute, the contents were admixed with a wood spatula. The contents were further sonicated for 9 minutes to solvate the CSD and stirred with a mechanical mixer for 3 minutes to dissolve the entire oxybutynin base. After the glass jar was rolled overnight to remove air, a liquid blend, containing dispersed CSD and no undissolved oxybutynin base crystals, was obtained. The blend was coated to a fluropolymer coated release liner Scotchpak® 1022, dried at room temperature for 4 min., and at 50°C for 90 min, or dried at room temperature for 4 min, at 40°C for 4 min and at 85°C for 15 minutes. A polyester backing film (Mediflex 1200, smooth polyester side) was laminated to the dry adhesive. The laminate was opaque and free of oxybutynin crystals immediately after it was made and remained opaque and free of oxybutynin crystals. Because the blend contained no undissolved oxybutynin base crystals and because the adhesive contact side of the backing film is a non-crystallization promoting polyester side and further because the silicone coated side of release liner (smooth fluropolymer coated side) is a non-crystallization promoting silicone coating on polyester, the laminate obtained was free of oxybutynin base crystals. Since the drug concentration in the adhesive matrix was higher than its solubility (3% by weight), the drug was supersaturated in the adhesive matrix. Thus, the adhesive matrix contained both dissolved oxybutynin base and undissolved oxybutynin base. In this example, the undissolved oxybutynin base was present in amorphous form dispersed in the adhesive matrix. The laminate is an amorphous drug-in-adhesive matrix laminate. Microscopic observation indicates there were no crystals present in the adhesive matrix.

Examples 5 to 9: Preparation of amorphous drug-in-polysiloxane adhesive matrix with a solvent method

The laminates of Example 5 to 9 were prepared similarly to Example 4. The adhesive matrix was sandwiched between the smooth polyester side of the backing Mediflex® and release liner Scotchpak™ 1022. The laminates of Example 4 to Example 9 were opaque and free of crystals.

**Table 2. Compositions of PIB based dry adhesive matrices**

| | Weight Percent | | | | | | | |
|---|---|---|---|---|---|---|---|---|
| Example # | 10¹ | 11¹ | 12¹ | 13² | 14³ | 15 | 16³ | 17 |
| Oxybutynin base | 17.5 | 17.5 | 17.5 | 23.81 | 19.08 | 17.5 | 14.80 | 17.5 |
| PIB | 31.59 | 31.14 | 30.68 | 50.00 | 32.95 | 38.75 | 37.77 | 45.5 |
| Polybutene | 37.91 | 37.36 | 36.82 | 21.43 | 37.90 | 38.75 | 44.14 | 0 |
| 1,3-butylene glycol | 3 | 3 | 3 | 1.90 | 0.92 | 0 | 0 | 0 |
| Dipropylene glycol | 5 | 0 | 5 | 2.86 | 0.97 | 0 | 0 | 0 |
| Propylene glycol | 0 | 6 | 2 | 0 | 0 | 0 | 0 | 32 |
| Mineral Oil | 0 | 0 | 0 | 0 | 0 | 0 | 0 | 32 |
| CSD | 5 | 5 | 5 | 0 | 8.18 | 5 | 3.20 | 5 |
| Total | 100 | 100 | 100 | 100 | 100 | 100 | 100 | 100 |

| | | | | | | | | |
|---|---|---|---|---|---|---|---|---|
| ¹Under the drying conditions described in the text for examples 8 to 10, 40% to 50% 1,3-butylene, 40% to 50% dipropylene glycol, and 40% to 50% propylene glycol were lost as indicated by GC analysis of the dry laminates. ²Under the drying condition described in the text for example 11, 50% to 60% 1,3-butylene and 50% to 60% dipropylene glycol were lost as indicated by GC analysis of the dry laminates. ³The amount of 1,3-butylene and the amount of dipropylene glycol present in the dried laminates were the actual amounts obtained by GC. | | | | | | | | |

Example 10: Preparation of crystalline drug-in-PIB adhesive matrix with a solvent method

In this example, 49.95 grams of polyisobutene solution (25.3% polyisobutene dissolved in heptane, the ratio of high molecular weight ("HMW") polyisobutene of average molecular weight 1,200,000 to low molecular weight ("LMW") polyisobutene average molecular weight 350,000 is 55/45) and 15.16 grams of polybutene, 1.20 grams of 1,3-butene glycol and 2.00 grams of dipropylene glycol were added to a jar. 2.00 grams of colloidal silicon dioxide was added under stirring. 7.00 grams of micronized oxybutynin base and an additional 36.98 grams of heptane were added. After the mixture was mixed, the jar was rolled overnight to remove trapped air. A liquid blend, containing dispersed CSD and undissolved oxybutynin base crystals, was obtained. The blend was coated to a silicone coated release liner, dried at room temperature for 5 min. and at 500°C for 90 minutes. A polyester backing film (Mediflex® 1200, smooth polyester side) was laminated to the dry adhesive. The laminate was clear right after it was made and became opaque containing dense fine crystals. The crystals were observed visually and microscopically. A three-layer patch is made by die-cutting the laminate. The drug-in-adhesive matrix is sandwiched between a backing film and a release liner. The composition of the dry adhesive matrix is described in table 2. Because the drug concentration in the adhesive matrix was higher than its solubility (3% by weight), the drug was supersaturated in the adhesive matrix. Thus, the PIB adhesive matrix contained both dissolved oxybutynin base and undissolved oxybutynin base. In this example, the undissolved oxybutynin base was present in crystalline form in the PIB matrix. The patch was a crystalline drug-in-adhesive matrix patch. In example 8, the oxybutynin base crystals were not completely dissolved in the blend containing heptane, in which oxybutynin base has a low solubility. Therefore, the blend contained dissolved oxybutynin base and undissolved oxybutynin base crystals, i.e. the blend is a saturated solution of oxybutynin base. The undissolved oxybutynin base crystals, which constituted crystallization seeds, promoted fast recrystallization of dissolved oxybutynin base after the solvent was removed. Because the drying temperature (500°C) was lower than the melting point of oxybutynin base crystals, crystals carried over from the blend and crystals formed during the drying process were not melted.

Examples 11 and 12: Preparation of crystalline drug-in-PIB adhesive matrix with a solvent method

The adhesive matrices of examples 11 and 12 were prepared similarly to example 10. The compositions of examples 11 and 12 matrices are described in Table 2.

Example 13: Crystallization of dissolved drug in adhesive matrix by heating

The laminate of this example was prepared similarly to the example 10 laminate. After the adhesive was coated on release liner Medirelease® 2249, the adhesive matrix containing undissolved oxybutynin base crystals was dried at room temperature for 5 minutes, and for 12 minutes at 85°C. A smooth backing film Mediflex® 1200 polyester side was laminated to the adhesive. The laminate was clear and free of crystals in the beginning, but dense crystals formed a month after it was prepared. This indicated that if the drug is not completely dissolved in the wet adhesive blend, heating at a temperature even above the drug's melting point will not evenly disperse the drug molecules as fine particles in an adhesive matrix or form a stable amorphous drug-in-adhesive matrix.

Example 14: Preparation of amorphous drug-in-polyisobutene adhesive matrices with a solvent method

In this example 119.02 grams of polyisobutene (25% polyisobutene dissolved in heptane, the ratio of high molecular weight polyisobutene of average molecular weight ("MW") 1,200,000 to low molecular weight polyisobutene average MW 350,000 is 55/45), 34.77 grams of polybutene, 6.00 grams of 1,3-butene glycol and 4.00 grams of dipropylene glycol were added to a jar. 7.50 Grams of colloidal silicon dioxide was added under stirring. 17.50 grams of micronized oxybutynin base and 96.59 grams of ethyl acetate and 27.12 grams of additional heptane were added. After the mixture was mixed and the jar was rolled overnight to remove trapped air, a liquid blend, containing dispersed CSD and no undissolved oxybutynin base crystals, was obtained. The blend was coated to a silicone coated Medirelease® 2249 release liner, dried at room temperature for 4 minutes and at 50°C for 90 minutes; or at room temperature for 4 minutes, at 85°C for 15 minutes, and at 400°C for 4 minutes. A polyester backing film (Mediflex® 1200, smooth polyester side) was laminated to the dry adhesive. The laminate was clear and free of oxybutynin crystals after it was made and remained clear and free of oxybutynin crystals. Because the blend contained no undissolved oxybutynin base crystals seeding recrystallization after the solvent was removed, and because the adhesive contact side of the backing film (smooth polyester side) and the adhesive contact side of release liner (smooth silicone coated side) were non-crystalline promoting, the laminate obtained was free of oxybutynin base crystals. Because the drug concentration in the adhesive matrix was higher than its solubility (3% by weight), the drug was supersaturated in the adhesive matrix. Thus, the PIB adhesive matrix contained both dissolved oxybutynin base and undissolved oxybutynin base. In this example, the undissolved oxybutynin base was present in amorphous form in the PIB matrix. The laminate is an amorphous drug-in-adhesive matrix laminate. Microscopical observation indicates there were no crystals present in the adhesive matrix.

Examples 15 and 17 are prepared similar to Example 14. In these examples, the laminate is an amorphous drug-in-adhesive matrix.

Example 16: Preparation of amorphous drug-in-PIB adhesive with hot melt extrusion method

In this example the adhesive matrix was prepared with a hot melt extrusion method. The extrusion temperature was 1100°C at which both the PIB, polybutene and oxybutynin base were melted. CSD and the glycols were dispersed in the adhesive. The entire oxybutynin base was dissolved or dispersed in the adhesive matrix at molecular level at the extrusion temperature. A thin film of the matrix was extruded to a smooth release liner and laminated with a smooth release liner.

The laminate obtained in Example 16 was clear and free of oxybutynin base crystals and remained clear and free of oxybutynin base crystals. Because the drug concentration in the adhesive matrix was higher than its solubility (3% by weight), the drug was supersaturated in the adhesive matrix. Thus, the PIB adhesive matrix contained both dissolved oxybutynin base and undissolved oxybutynin base. In this example, the undissolved oxybutynin base was present in amorphous form dispersed in the PIB matrix. The laminate is an amorphous drug-in-adhesive matrix laminate. Microscopical observation indicates there were no crystals present in the adhesive matrix.

**In Vitro Flux Data**

In vitro flux studies were performed using Franz cells and Human Cadaver Epidermis in incubators at 32°C. Oxybutynin base penetrated through the skin into the receptor phase at several different time points up to 96 hours. Analysis was performed by HPLC. Each study consisted of a different skin donor and 4 replicates per donor.

The results of the 96 hour cumulative flux through human cadaver epidermis is described in Table 3. The flux of amorphous oxybutynin base-in-adhesive matrices is about 250% to about 600% times greater than the flux of crystalline oxybutynin base-in-adhesive matrices.

**Table 3. Cumulative skin (human cadaver epidermis) flux at 96 hours**

| | Drug form | Initial time point.Cumulative skin flux, µg/cm² | After 3 months at 40°C Cumulative skin flux, µg/cm² |
|---|---|---|---|
| Example 18 | Crystalline drug-in-polysiloxane adhesive, same as in Example #1 | 78¹ | - |
| Example 19 | Amorphous drug-in-polysiloxane adhesive, same as in Example #4 | 210¹ | - |
| Example 20 | Amorphous drug-in-polysiloxane adhesive, same as in Example #5 | 213² | 218¹ |
| Example 21 | Amorphous drug-in-polysiloxane adhesive, same as in Example #6 | 203¹ | - |
| Example 22 | Amorphous drug-in-polysiloxane adhesive, same as in Example #7 | 212³ | 219¹ |
| Example 23 | Crystalline drug-in-PIB adhesive, same as in Example #10 | 61¹ | - |
| Example 24 | Crystalline drug-in-PIB adhesive, same as in Example #11 | 57¹ | - |
| Example 25 | Crystalline drug-in-PIB adhesive, same as in Example #12 | 35¹ | - |
| Example 26 | Amorphous drug-in-PIB adhesive, same as in Example #14 | 199¹ | - |
| Example 27 | Amorphous drug-in-PIB adhesive, same as in Example #16 | 179¹ | 198¹ |
| Example 28 | Amorphous drug-in-PIB/mineral oil adhesive, same as in Example #17 | 246¹ | - |

**Table 4. Smoothness by Gurley 4340 Automatic Densometer**

| Backing Film | Inner side | | Outer side | |
|---|---|---|---|---|
| | Smoothness (standard Gurley seconds) | Chemistry | Smoothness (standard Gurley seconds) | Chemistry |
| Mediflex® 1000 | 13360 | LLDPE/LDPE | 13397 | LLDPE/LDPE |
| 3M Cotran™ 9722 | 21620 | Polyolefin | 12555 | polyolefin |
| Mediflex® 1201 | 7240 | PET | 17871 | PE/9% EVA |
| Mediflex® 1200 | 20719 | PET | 14245 | PE/9% EVA |
| Dow Chemical BLF 2050 | 16783 | EVA/Olefin | 17620 | EVA/Olefin |

Table 4 includes data of backing film surface smoothness measured with a Gurley 4340 Densometer. Table 4 also indicates the different chemical compositions of various backing film surfaces. The inventors of the present invention found that amorphous oxybutynin base-in-silicone adhesive matrices did not crystallize if the matrix was sandwiched between a smoother Mediflex® 1200 polyester side and Scotchpak™ 1022. However, crystals formed if the same amorphous oxybutynin base-in-adhesive matrix was sandwiched between a rougher Mediflex® 1000 polyolefin/EVA copolymer side and Scotchpak™ 1022.

**Table 5: Effect of backing film surfaces on crystallization of amorphous oxybutynin base-in-adhesive matrix**

| Example | Adhesive matrix composition same as in | Backing Film | Release liner | Amorphous Oxybutynin Base-in Adhesive between backing and liner in laminate |
|---|---|---|---|---|
| Example 29 | Example 4 | Mediflex® 1200, shiny polyester side, smooth | Scotchpak™ 1022 fluropolymer side, smooth | Free of crystals |
| Example 30 | Example 5 | Mediflex® 1200, shiny polyester side, smooth | Scotchpak™ 1022 fluropolymer side, smooth | Free of crystals |
| Example 31 | Example 6 | Mediflex® 1200, shiny polyester side, smooth | Scotchpak™ 1022 fluropolymer side, smooth | Free of crystals |
| Example 32 | Example 7 | Mediflex® 1200, shiny polyester side, smooth | Scotchpak™ 1022 fluropolymer side, smooth | Free of crystals |
| Example 33 | Example 5 | Dupont polyester type S, smooth | Scotchpak™ 1022 fluropolymer side, smooth | Free of crystals |
| Example 34 | Example 7 | Dupont polyester type S, smooth | Scotchpak™ 1022 fluropolymer side, smooth | Free of crystals |
| Example 35 | Example 5 | Mediflex® 1200, matte EVA side, rough | Scotchpak™ 1022 fluropolymer side, smooth | Crystals formed in about a week |
| Example 36 | Example 7 | Mediflex® 1200, matte EVA side, rough | Scotchpak™ 1022 fluropolymer side, smooth | Crystals formed in about a week |
| Example 37 | Example 7 | Mediflex® 1000, shiny polyethylene side, smoother than the another matte side | Scotchpak™ 1022 fluropolymer side, smooth | Crystals formed in about two weeks |
| Example 38 | Example 7 | Mediflex® 1000, matte polyethylene side, rougher than other side | Scotchpak™ 1022 fluropolymer side, smooth | Crystals in about two weeks |
| Example 39 | Example 8 | Cotran™ 9722, as smooth as Mediflex® 1200 polyester side | Scotchpak™ 1022 fluropolymer side, smooth | Crystals form in about two weeks |
| Example 40 | Example 8 | Mediflex® 1201, rougher than Mediflex® 1200 | Scotchpak™ 1022 fluropolymer side | Free of crystals |
| Example 41 | Example 8 | Mediflex® 1200, heavily wrinkled by hand | Scotchpak™ 1022 fluropolymer side | Free of crystals |
| Example 42 | Example 7 | Dow BLF 2050 | Scotchpak™ 1022 fluropolymer side | Free of crystals |
| Example 43 | Example 8 | Dow Corning Silicone membrane 7-4107 | Scotchpak™ 1022 fluropolymer side | Free of crystals |
| Example 44 | Example 8 | Dow Corning silicone coating on Mediflex® 1000 | Scotchpak™ 1022 fluropolymer side | Free of crystals |
| Example 45 | Example 8 | Silicone 7-4302/CSD placebo layer on Mediflex® 1000 | Scotchpak™ 1022 fluropolymer side | Free of crystals |
| Example 46 | Example 14 | Mediflex® 1200 polyester side | Medirelease® 2249 | Free of crystals |
| Example 47 | Example 16 | Mediflex® 1000 | Medirelease® 2249 | Crystal formed in about two weeks |

Table 5 includes data which indicates that the surface chemical composition of a liner or film affects crystallization. No matter how rough the polyester backing films(Mediflex® 1200 and Mediflex® 1201) were, crystallization did not occur on amorphous drug-in-adhesive matrices sandwiched between a polyester backing film and a Scotchpak™ 1022 release liner. However, no matter how smooth the polyolefin film or polyethylene backing films were (Mediflex® 1000 and 3M Cotran™ 9722), crystals formed in the amorphous oxybutynin base-in-adhesive matrix sandwiched between a polyolefin backing film and Scotchpak™ 1022 release liner. As such, the results of testing indicate that the polyester backing films do not have the proper nuclei or seeding particles to initiate oxybutynin base crystallization. Polyolefin and olefin/low level EVA copolymer backing films, however, do have the proper nuclei to initiate oxybutynin crystallization.

Moreover, crystallization did not occur when the same amorphous oxybutynin base-in-silicone adhesive matrix was laminated between a fluropolymer coated release liner (such as Scotchpak™ 1022) and a non-polyolefin backing film (such as Dupont polyester type S film), a silicone coated polyolefin backing film, or a Bio-PSA 7-4302/Colloidal silicon dioxide placebo layer.

Similarly, crystallization of oxybutynin base occurred when an amorphous oxybutynin base-in-PIB adhesive matrix was laminated between a silicon release liner and polyolefin backing film such as the Mediflex® 1000 and olefin/EVA side of Mediflex 1200. But crystallization did not occur when the same amorphous oxybutynin base-in-PIB adhesive matrix was laminated between a silicone coated release liner and a polyester backing film such as the polyester side of Mediflex® 1200.

**Table 6. Effect of heat-curing die-cut patches on stability of amorphous form of oxybutynin base in polysiloxane matrices (all the patches used backing Mediflex® 1200 smooth polyester side and Scotchpak® 1022 release liner)**

| Example No. | Matrix composition same as in | Release liner size | Heat curing at 85°C for 15 min | 1 Month¹ at 20°C or 40°C | 3 Month² at 20°C or 40°C) | After 10 cycles of Freeze and Thaw Stability |
|---|---|---|---|---|---|---|
| Example 48 | Example 5 | Same size as the adhesive matrix layer. No peeling off release liner, no back slitting | No | Crystals observed on edge of patch | more crystals observed than 1 month | Crystals observed on edge of patch |
| Example 49 | Example 5 | Same size as the adhesive matrix layer. No peeling off release liner, no back slittings | Yes | No crystals observed | No crystals observed in patches from newly opened pouches. But some crystals observed from patches opened in earlier months. | No crystal observed |
| Example 50 | Example 5 | Peeling off liner and replaced with an oversized liner | No | Crystals formed both on edge and randomly on center of patch | - | Crystals formed both on edge and randomly on center of patch |
| Example 51 | Example 5 | Peeling off the liner and replaced with an oversized liner, with or without back slitting | Yes | No crystal observed | No crystal observed | No crystal observed |
| Example 52 | Example 7 | Same size as the adhesive matrix layer. No peeling off release liner, no back slitting | No | Crystals observed on edge of patch | More crystals observed than month 1 | Crystals observed on edge of patch |
| Example 53 | Example 7 | Same size as the adhesive matrix layer. No peeling off release liner, no back slitting | Yes | No crystals observed | No crystals observed in patches from newly open pouches. But some crystals observed from patches opened in earlier months | No crystal observed |
| Example 54 | Example 7 | Peeling off the liner and replaced with an oversized liner | No | Crystals formed both on edge and randomly on center of patch | - | Crystals observed from both edge and center of patch |
| Example 55 | Example 7 | Peeling off the liner and replaced with an oversized liner, with or without back slitting | Yes | No crystals observed | No crystals observed | No crystal observed |

| | | | | | | |
|---|---|---|---|---|---|---|
| ¹ One month after patches were die-cut and pouched and stored at 22°C and 40°C. ² Tree months after patched were die-cut and pouched and stored at 22°C and 40°C. | | | | | | |

As shown in Tables 6 and 7, it has been discovered that die-cutting destabilizes the amorphous form of oxybutynin base in an adhesive matrix. As such, crystallization occurred on the edge of patch and progressed towards the center. It has also been discovered that delamination (peeling off the release liner and replacing it with the same release liner or with a new piece of release liner) destabilized the amorphous form of oxybutynin base in adhesive matrix. Thus, crystals formed in about a 5% area of the patch over three weeks. Moreover, the crystals formed randomly in the delaminated patch. The amount of crystals and rate of crystallization were dependent on the peeling force.

It has also been discovered that heat-curing die-cut patches, with or without back slitting and with or without delamination restabilizes the amorphous form of oxybutynin base in adhesive matrices and prevents the amorphous drug from crystallizing.

In Tables 6 and 7, the stability of heat cured patches were compared to the stability of the patches with an oversized release liner that were not heat-cured. It was discovered that heat curing at a temperature above the melting point of oxybutynin base (about 56°C) for 15 minutes, restabilized the amorphous form of oxybutynin base in an adhesive matrix. Thus, no crystal growth was observed even after the heat-cured patches were aged for 3 months at room temperature or 40°C for the polysiloxane based adhesive matrices.

When comparing the stability of heat-cured patches in which the release liner is of the same size as the adhesive layer, it was found that no crystals were observed for patches newly removed from pouches at each of the time points (1, 2, 3, 6, 8 months) for microscopy observation. However, crystals were observed on the patches that were removed from pouches at earlier time points because the adhesive edge was in contact on the pouch and the process of removing the patch from the pouch damaged the edge of adhesive. As such, damaging the edge of adhesive initiated crystallization. However, use of an oversized release liner in contact with the adhesive layer prevented the edge of adhesive from contacting the pouch. Moreover, when an oversized released liner was used in combination with heat curing, crystallization was prevented during the handling process. In addition, crystal formation of heat-cured patches did not occur after 10 cycles of freezing and thawing. These results indicate the amorphous form of oxybutynin base in an adhesive matrix of an undie-cut laminate is stable for a sufficient period of time. The results also indicate the amorphous form of oxybutynin base in an adhesive matrix in a heat-cured die-cut patch, with or without back slitting and with or without delamination, is stable for a sufficient period of time.

**Table 7. Effect of heat-curing die-cut patches on stability of amorphous form of oxybutynin base in PIB matrices (backing Mediflex® 1200 and release liner Medirelease® 2249)**

| | Matrix composition | Release liner size | Heat curing at 85°C for 15 min | 1 month¹ at 20°C or 40°C | 3 month² at 20°C or 40°C | After 4 cycles of Freeze and Thaw Stability |
|---|---|---|---|---|---|---|
| Example 56 | Example 12 | Same size as the adhesive matrix layer. No peeling off release liner | No | Crystals observed on edge of patch | More crystals observed | crystals observed |
| Example 57 | Example 12 | Replaced the original liner with an oversized liner | Yes | No crystals observed | No crystals observed | No crystals observed |
| Example 58 | Example 14 | Same size as the adhesive matrix layer. No peeling off release liner | No | Crystals observed on edge of patch | More crystals observed | Crystals Observed |

Polarized light microscopy analysis was performed with an Olympus BX51. Differential scanning Calorimetry ("DSC") and Modualted DSC were performed with a TA Q-1000 DSC instrument, and were used to characterize the amorphous oxybutynin base-in-adhesive matrix, and to determine whether crystals were present in the laminates and patches.

The DSC of the crystalline oxybutynin base powder showed a sharp endothermic melting peak at about 56°C and no glass transition temperature between -90 to 80°C. The melt in the DSC pan obtained from the first run was rapidly cooled to -90°C, and then the temperature was ramped from about -90°C to about 80°C. This melt showed a glass transition temperature at about -20°C, but no endothermic melting peak, indicating the melt was in amorphous form. The DSC of crystalline oxybutynin base-in-silicone adhesives showed an endothermic melting peak and a Tg at about -120°C, which is believed to be the Tg of the silicone adhesive containing some dissolved oxybutynin base. The DSC of amorphous oxybutynin base-in-silicone adhesive showed a Tg at about -120°C, which is believed to be the Tg of a silicone adhesive containing dissolved oxybutynin base, and a Tg at about -20°C, which is believed to be the Tg of dispersed amorphous oxybutynin base. For patches that were not heat cured, crystals were observed visually and by microscopy on the edge of patch. DSC of the partially crystallized patches showed two glass transition temperatures (at -120 and -20°C regions, respectively) and an endothermic melt peak. This is consistent with the presence of both crystalline oxybutynin base and amorphous oxybutynin base in the partially crystallized patches.

Example 59 (transdermal delivery device comprising scopolamine as an active agent): 23.83 grams of ethyl acetate were added to 4 grams of scopolamine free base. The solution was stirred until the scopolamine crystals were completely dissolved. To this solution, 92.31 grams of PIB solution in heptane (25% polymer solid) was added. The admixture was mixed with a propeller at high speed for 3 minutes to form a uniform solution. However, after the solution was rolled over for 1 hour, many crystals formed. Thus, 27.11 grams of more ethyl acetate were added to completely dissolve the scopolamine crystals and form a uniform solution. The solution remained to be clear after it was rolled overnight to remove trapped air. The viscous solution was coated to a release liner Medirelease® 2249, dried in an over to remove solvent, laminated to backing film Mediflex® 1200 polyester side. A crystal free laminate was formed.

Example 60 (transdermal delivery device comprising naltrexone as an active agent): To a jar containing 3.15 grams of naltrexone and 0.63 grams of colloidal silicon dioxide, 24.5 grams of ethanol was added. The admixture was mixed and heated to 44°C in a sonicator to form a brownish hazy solution. All of the naltrexone crystals were dissolved but colloidal silicon dioxide was dispersed. After the hazy solution was cooled down to about 30°C, 38.16 grams of Duro-Tak 87-2979 (45.12% polymer solid) was added. The admixture was mixed with a propeller at high speed for 3 minutes to result in a uniform hazy solution. Again, all of the naltrexone was dissolved but colloidal silicon dioxide was dispersed. After it was rolled overnight to remove trapped air, the solution was coated to release liner Mediflex® 2249 and dried in an oven. The adhesive side on release liner was laminated to backing film Mediflex® 1200. The resulting laminate was free of naltrexone crystals.

Example 61 (transdermal delivery device comprising naltrexone as an active agent): To 3.55 grams of naltrexone and 0.71 grams of colloidal silicone dioxide in a glass jar, 8.33 grams of ethanol was added. The admixture was mixed and heated to about 50°C in a water bathed sonicator. After a hazy solution was formed (again, all of the naltrexone crystals were dissolved and colloidal silicon dioxide was dispersed), the solution was cooled down to about 30°C. 34 Grams of Bio-PSA 7-4302 was added. The admixture was mixed with a propeller at high speed for 2 minutes to result in a uniform solution (only colloidal silicon dioxide was dispersed and all other ingredients were dissolved). After the solution was rolled overnight to remove trapped air, it was coated to release liner Scotchpak® 1022 and dried in an oven. Backing film Mediflex® was laminated to the adhesive side. The laminate formed was free of naltrexone crystals.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

The present application also includes the following numbered clauses:
Clause 1. A transdermal delivery device comprising:
   a) a backing layer,
   b) an adhesive matrix layer comprising a supersaturated concentration of at least one active agent substantially in amorphous form within said adhesive matrix, and
   c) a release liner.
Clause 2. The transdermal delivery device of clause 1, wherein said active agent is present in an amount of about 0.1% to about 50% by weight of said adhesive matrix.
Clause 3. The transdermal delivery device of clause 2, wherein said active agent is present in an amount of about 1% to about 20% by weight of said adhesive matrix.
Clause 4. The transdermal delivery device of clause 1, wherein the concentration of said active agent is from about 0.1% to about 10000% above the solubility of said active agent in said adhesive matrix.
Clause 5. The transdermal delivery device of clause 4, wherein the concentration of said active agent is from about 5% to about 5000% above the solubility of said active agent in said adhesive matrix.
Clause 6. The transdermal delivery device of clause 4, wherein the concentration of said active agent is from about 10% to about 1000% above the solubility of said active agent in said adhesive matrix.
Clause 7. The transdermal delivery device of clause 1, wherein said backing layer and said release liner are substantially non-crystallization inducing and free of crystallization nuclei or crystallization seeding particles.
Clause 8. The transdermal delivery device of clause 7, wherein said backing layer is selected from the group consisting of polyester films, polyethelene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.
Clause 9. The transdermal delivery device of clause 7, wherein said backing layer is the same size as said adhesive matrix layer.
Clause 10. The transdermal delivery device of clause 7, wherein said backing layer is larger than said adhesive matrix layer.
Clause 11. The transdermal delivery device of clause 7, wherein said release liner is selected from the group consisting of polyester liners, polyurethane liners, polyester liners with a silicone coating, polyurethane liners with a silicone coating, polyester liners with a fluorosilicone coating, polyurethane liners with a fluorosilicone coating, silicon coated polyester liners, silicon coated polyurethane liners, polyester liners with a fluoropolymer coating, and polyurethane liners with a fluoropolymer coating.
Clause 12. The transdermal delivery device of clause 11, wherein said release liner is larger than said adhesive matrix layer.
Clause 13. The transdermal delivery device of clause 12, wherein said release liner is about 0.1mm to about 20mm larger than said adhesive matrix layer.
Clause 14. The transdermal delivery device of clause 1, wherein said adhesive matrix layer comprises an adhesive material selected from the group consisting of polyisobutylene, polysiloxane, acrylic adhesives, natural and synthetic rubber adhesives, and mixtures thereof.
Clause 15. The transdermal delivery device of clause 14, wherein said adhesive material is present in an amount of from about 50% to about 99% by weight of said adhesive matrix layer.
Clause 16. The transdermal delivery device of clause 15, wherein said adhesive material is present in an amount of from about 60% to about 90% by weight of said adhesive matrix layer.
Clause 17. The transdermal delivery device of clause 1, wherein said adhesive matrix layer further comprises one or more tackifiers.
Clause 18. The transdermal delivery device of clause 17, wherein said one or more tackifiers is selected from the group consisting of polybutenes, mineral oils, and polysiloxanes.
Clause 19. The transdermal delivery device of clause 1, wherein said adhesive matrix layer further comprises one or more cohesive enhancers.
Clause 20. The transdermal delivery device of clause 19, wherein said one or more cohesive enhancers is selected from the group consisting of colloidal silicone dioxide, zinc oxide, polyvinylpyrrolidone, polyvinylpyrrolidone-co-vinylacetate, acrylate copolymers, crosspovidone, bentonites, clays, ethyl cellulose and mixtures thereof.
Clause 21. The transdermal delivery device of clause 1, wherein said adhesive matrix layer further comprises one or more flux enhancers.
Clause 22. The transdermal delivery device of clause 21, wherein said one or more flux enhancers is selected from the group consisting of propylene glycol, butylene glycol, dipropylene glycol, diethylene glycol, propyl palmitate, isopropyl palmitate, propyl myristate, pendadecanol, octadecanol, propylene glycol monoesters, polyethylene glycol monoesters and glycerol monoesters.
Clause 23. The transdermal delivery device of clause 1, further comprising a drug release regulating membrane layer and a reservoir layer.
Clause 24. The transdermal delivery device of clause 23, wherein at least one of said drug release regulating membrane layer and said reservoir layer contains said active agent.
Clause 25. The transdermal delivery device of clause 1, further comprising an overlay layer in communication with said backing layer.
Clause 26. The transdermal delivery device of clause 25, wherein said overlay layer is larger than said backing layer.
Clause 27. The transdermal delivery device of clause 26, wherein said overlay layer is about 0.01mm to about 20mm larger than said backing layer.
Clause 28. A transdermal delivery device comprising:
   a) a backing layer,
   b) an adhesive matrix layer comprising a supersaturated concentration of oxybutynin substantially in amorphous form within said adhesive matrix, and
   c) a release liner.
Clause 29. The transdermal delivery device of clause 28, wherein said oxybutynin is present in an amount of about 0.1% to about 50% by weight of said adhesive matrix.
Clause 30. The transdermal delivery device of clause 29, wherein said oxybutynin is present in an amount of about 1% to about 20% by weight of said adhesive matrix.
Clause 31. The transdermal delivery device of clause 28, wherein the concentration of said oxybuytnin is from about 0.1% to about 10000% above the solubility of said oxybutynin in said adhesive matrix.
Clause 32. The transdermal delivery device of clause 31, wherein the concentration of said oxybutynin is from about 5% to about 5000% above the solubility of said oxybutynin in said adhesive matrix.
Clause 33. The transdermal delivery device of clause 31, wherein the concentration of said oxybutynin is from about 10% to about 1000% above the solubility of said oxybutynin in said adhesive matrix.
Clause 34. The transdermal delivery device of clause 28, wherein said backing layer and said release liner are substantially non-crystallization inducing and free of crystallization nuclei or crystallization seeding particles.
Clause 35. The transdermal delivery device of clause 34, wherein said backing layer is selected from the group consisting of polyester films, polyethelene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.
Clause 36. The transdermal delivery device of clause 34, wherein said backing layer is the same size as said adhesive matrix layer.
Clause 37. The transdermal delivery device of clause 34, wherein said backing layer is larger than said adhesive matrix layer.
Clause 38. The transdermal delivery device of clause 34, wherein said release liner is selected from the group consisting of polyester liners, polyurethane liners, polyester liners with a silicone coating, polyurethane liners with a silicone coating, polyester liners with a fluorosilicone coating, polyurethane liners with a fluorosilicone coating, silicon coated polyester liners, silicon coated polyurethane liners, polyester liners with a fluoropolymer coating, and polyurethane liners with a fluoropolymer coating.
Clause 39. The transdermal delivery device of clause 34, wherein said release liner is larger than said adhesive matrix layer.
Clause 40. The transdermal delivery device of clause 39, wherein said release liner is about 0.1mm to about 20mm larger than said adhesive matrix layer.
Clause 41. The transdermal delivery device of clause 28, wherein said adhesive matrix layer comprises an adhesive material selected from the group consisting of polyisobutylene, polysiloxane, acrylic adhesives, natural and synthetic rubber adhesives, and mixtures thereof.
Clause 42. The transdermal delivery device of clause 41, wherein said adhesive material is present in an amount of from about 50% to about 99% by weight of said adhesive matrix layer.
Clause 43. The transdermal delivery device of clause 42, wherein said adhesive material is present in an amount of from about 60% to about 90% by weight of said adhesive matrix layer.
Clause 44. The transdermal delivery device of clause 28, wherein said adhesive matrix layer further comprises one or more tackifiers.
Clause 45. The transdermal delivery device of clause 44, wherein said one or more tackifiers is selected from the group consisting of polybutenes, mineral oils, and polysiloxanes.
Clause 46. The transdermal delivery device of clause 28, wherein said adhesive matrix layer further comprises one or more cohesive enhancers.
Clause 47. The transdermal delivery device of clause 46, wherein said one or more cohesive enhancers is selected from the group consisting of colloidal silicone dioxide, zinc oxide, polyvinylpyrrolidone, polyvinylpyrrolidone-co-vinylacetate, acrylate copolymers, crosspovidone, bentonites, clays, ethyl cellulose and mixtures thereof.
Clause 48. The transdermal delivery device of clause 28, wherein said adhesive matrix layer further comprises one or more flux enhancers.
Clause 49. The transdermal delivery device of clause 48, wherein said one or more flux enhancers is selected from the group consisting of propylene glycol, butylene glycol, dipropylene glycol, diethylene glycol, propyl palmitate, isopropyl palmitate, propyl myristate, pendadecanol, pendadecalactone, octadecanol, propylene glycol monoesters, polyethylene glycol monoesters and glycerol monoesters.
Clause 50. The transdermal delivery device of clause 28, further comprising a drug release regulating membrane layer and a reservoir layer.
Clause 51. The transdermal delivery device of clause 50, wherein at least one of said drug release regulating membrane layer and said reservoir layer contains said oxybutynin.
Clause 52. The transdermal delivery device of clause 28, further comprising an overlay layer in communication with said backing layer.
Clause 53. The transdermal delivery device of clause 52, wherein said overlay layer is larger than said backing layer.
Clause 54. The transdermal delivery device of clause 53, wherein said overlay layer is about 0.01mm to about 20mm larger than said backing layer. I have temporarily renumbered this clause.
Clause 55. A transdermal delivery device comprising:
   a) a backing layer,
   b) an adhesive matrix layer comprising a supersaturated concentration of an active agent substantially in amorphous form within said adhesive matrix, and
   c) a release liner,
   wherein said active agent is selected from the group consisting of piroxicam, fentanyl, naltrexone, scopolamine and a steroid.
Clause 56. The transdermal delivery device of clause 55, wherein said steroid is selected from the group consisting of estrogens, progestogens, testosterone, noregestrel, norethindrone acetate, medroxyprogesterone acetate, levonorgestrel, and norelgestromin.
Clause 57. The transdermal delivery device of clause 55, wherein said active agent is present in an amount of from about 0.1% to about 50% by weight of said adhesive matrix.
Clause 58. The transdermal delivery device of clause 55, wherein said active agent is present in an amount of from about 1% to about 20% by weight of said adhesive matrix.
Clause 59. The transdermal delivery device of clause 55, wherein the concentration of said active agent is from about 0.1% to about 10000% above the solubility of said active agent in said adhesive matrix.
Clause 60. The transdermal delivery device of clause 55, wherein the concentration of said active agent is from about 5% to about 5000% above the solubility of said active agent in said adhesive matrix.
Clause 61. The transdermal delivery device of clause 55, wherein the concentration of said active agent is from about 10% to about 1000% above the solubility of said active agent in said adhesive matrix.
Clause 62. The transdermal delivery device of clause 55, wherein said backing layer and said release liner are substantially non-crystallization inducing and free of crystallization nuclei or crystallization seeding particles.
Clause 63. The transdermal delivery device of clause 55, wherein said backing layer is selected from the group consisting of polyester films, polyethelene films, metal films, metalized polyester films, nylon films, ethylene vinyl acetate film laminated to a polyester, ethylene vinyl acetate films laminated to a metalized polyester, polyvinylidene fluoride films, silicone coated polyester films, silicone coated polyolefin films, and silicone coated ethyl vinyl acetate films.
Clause 64. The transdermal delivery device of clause 62, wherein said backing layer is the same size as said adhesive matrix layer.
Clause 65. The transdermal delivery device of clause 62, wherein said backing layer is larger than said adhesive matrix.
Clause 66. The transdermal delivery device of clause 62, wherein said release liner is selected from the group consisting of polyester liners, polyurethane liners, polyester liners with a silicone coating, polyurethane liners with a silicone coating, polyester liners with a fluorosilicone coating, polyurethane liners with a fluorosilicone coating, silicon coated polyester liners, silicon coated polyurethane liners, polyester liners with a fluoropolymer coating, and polyurethane liners with a fluoropolymer coating.
Clause 67. The transdermal delivery device of clause 62, wherein said release liner is larger than said adhesive matrix layer.
Clause 68. The transdermal delivery device of clause 67, wherein said release liner is about 0.1mm to about 20mm larger than said adhesive matrix layer.
Clause 69. The transdermal delivery device of clause 55, wherein said adhesive matrix layer comprises an adhesive material selected from the group consisting of polyisobutylene, polysiloxane, acrylic adhesives, natural and synthetic rubber adhesives, and mixtures thereof.
Clause 70. The transdermal delivery device of clause 69, wherein said adhesive material is present in an amount of from about 50% to about 99% by weight of said adhesive matrix layer.
Clause 71. The transdermal delivery device of clause 70, wherein said adhesive material is present in an amount of from about 60% to about 90% by weight of said adhesive matrix layer.
Clause 72. The transdermal delivery device of clause 55, wherein said adhesive matrix layer further comprises one or more tackifiers.
Clause 73. The transdermal delivery device of clause 72, wherein said one or more tackifiers is selected from the group consisting of polybutenes, mineral oils, and polysiloxanes.
Clause 74. The transdermal delivery device of clause 55, wherein said adhesive matrix layer further comprises one or more cohesive enhancers.
Clause 75. The transdermal delivery device of clause 74, wherein said one or more cohesive enhancers is selected from the group consisting of colloidal silicone dioxide, zinc oxide, polyvinylpyrrolidone, polyvinylpyrrolidone-co-vinylacetate, acrylate copolymers, and crosspovidone, bentonites, clays, ethyl cellulose and mixtures thereof.
Clause 76. The transdermal delivery device of clause 55, wherein said adhesive matrix layer further comprises one or more flux enhancers.
Clause 77. The transdermal delivery device of clause 76, wherein said one or more flux enhancers is selected from the group consisting of propylene glycol, butylene glycol, dipropylene glycol, diethylene glycol, propyl palmitate, isopropyl palmitate, propyl myristate, and glycerol monoesters.
Clause 78. The transdermal delivery device of clause 55, further comprising a drug release regulating membrane layer and a reservoir layer.
Clause 79. The transdermal delivery device of clause 78, wherein at least one of said drug release regulating membrane layer and said reservoir layer contains said active agent.
Clause 80. The transdermal delivery device of clause 55, further comprising an overlay layer in communication with said backing layer.
Clause 81. The transdermal delivery device of clause 80, wherein said overlay layer is larger than said backing layer.
Clause 82. The transdermal delivery device of clause 81, wherein said overlay layer is about 0.01mm to about 20mm larger than said backing layer.
Clause 83. A method of preparing an adhesive matrix containing at least one active agent that is supersaturated and present in amorphous form comprising:
   a) dissolving said at least one active agent and an adhesive material in a solvent in an amount so as to provide said at least one active agent at a subsaturated concentration in an adhesive matrix solution,
   b) casting said subsaturated active agent in said adhesive matrix solution to one of a release liner and a backing layer,
   c) removing said solvent at a temperature which is at, below, or above the melting point of said at least one active agent to form a dry adhesive matrix in which said at least one active agent is in a supersaturated concentration, and
   d) laminating the other of said release liner and said backing film to said supersaturated active agent in said dry adhesive matrix, so that said supersaturated active agent in said dry adhesive matrix is between said release liner and said backing layer.
Clause 84. The transdermal delivery device of clause 83, where said at least one active agent is selected from the group consisting of oxybutynin, piroxicam, fentanyl, naltrexone, scopolamine and a steroid.
Clause 85. The method of clause 83, wherein said release liner and said backing layer are non-crystallization inducing and free of crystallization nuclei or crystallization seeding particles.
Clause 86. The method of clause 83, wherein said supersaturated active agent in said adhesive matrix further comprises one or more additives which are dispersed as liquid or solid particles in said dry adhesive matrix.
Clause 87. The method of clause 86, wherein said one or more additives are selected from the group consisting of penetration enhancers, crystal growth inhibitors, tackifiers, cohesive enhancers, plasticizers, and antioxidants.
Clause 88. The method of clause 86, wherein said one or more additives are present in an amount of from about 1% to about 50% by weight of said adhesive matrix.
Clause 89. The method of clause 88, wherein said one or more adhesives are present in an amount of from about 2% to about 25% by weight of said adhesive matrix.
Clause 90. The method of clause 83, wherein said solvent is present in an amount of from about 1% to about 200% more than the amount necessary to solubilize said active agent and said adhesive.
Clause 91. The method of clause 83, wherein said solvent is selected from the group consisting of heptane, ethyl acetate, toluene, xylene, ethanol, and isopropanol.
Clause 92. The method of clause 84, wherein said active agent is present in an amount of from about 0.1% to about 50% by weight of said adhesive matrix layer.
Clause 93. The method of clause 92, wherein said active agent is present in an amount of from about 1% to about 20% by weight of said adhesive matrix layer.
Clause 94. The method of clause 83, wherein said adhesive matrix is present in an amount of from about 50% to about 99% by weight of said adhesive matrix layer.
Clause 95. The method of clause 94, wherein said adhesive matrix is present in an amount of from about 60% to about 90% by weight of said adhesive matrix layer.
Clause 96. A method of preparing an adhesive matrix containing at least one active agent that is supersaturated and present in amorphous form comprising:
   a) admixing said at least one active agent with an adhesive matrix at a supersaturated concentration,
   b) heating said supersaturated concentration of said at least one active agent in said adhesive matrix to a temperature which allows said at least one active agent to be completely dissolved and uniformly dispersed in said adhesive matrix to create a hot melt,
   c) casting said hot melt to one of a release liner and a backing layer, at a predetermined temperature, and
   d) laminating the other of said release liner and said backing layer to said hot melt, so that said hot melt
   is between said release liner and said backing layer.
Clause 97. The transdermal delivery device of clause 96, wherein said at least one active agent is selected from the group consisting of oxybutynin, piroxicam, fentanyl, naltrexone, scopolamine and a steroid.
Clause 98. The method of clause 96, wherein said release liner and said backing layer are non-crystallization inducing and free of crystallization nuclei or crystallization seeding particles.
Clause 99. The method of clause 96, wherein said hot melt further comprises one or more additives which are dispersed in said adhesive matrix.
Clause 100. The method of clause 99, wherein said one or more additives are selected from the group consisting of penetration enhancers, crystal growth inhibitors, tackifiers, cohesive enhancers, plasticizers, and antioxidants.
Clause 101. The method of clause 99, wherein said one or more additives are present in an amount of from about 1% to about 50% by weight of said adhesive matrix layer.
Clause 102. The method of clause 101, wherein said one or more adhesives are present in an amount of from about 2% to about 25% by weight of said adhesive matrix layer.
Clause 103. The method of clause 96, wherein said active agent is present in an amount of from about 0.1% to about 50% by weight of said adhesive matrix layer.
Clause 104. The method of clause 103, wherein said active agent is present in an amount of from about 1% to about 20% by weight of said adhesive matrix layer.
Clause 105. The method of clause 96, wherein said adhesive matrix is present in an amount of from about 50% to about 99% by weight of said adhesive matrix layer.
Clause 106. The method of clause 105, wherein said adhesive matrix is present in an amount of from about 60% to about 90% by weight of said adhesive matrix layer.
Clause 107. A method of removing crysllization nuclei and reestablishing the favored internal adhesive matrix environment of a transdermal drug delivery device having a backing layer, an adhesive matrix layer having a supersaturated concentration of an active agent substantially in the amorphous form within said adhesive matrix layer, and a release liner, said method comprising curing said transdermal delivery device.
Clause 108. The method of clause 107, wherein said active agent is selected from the group consisting of oxybutynin, piroxicam, fentanyl, naltrexone, scopolamine and a steroid.
Clause 109. The method of clause 107, wherein said curing comprises heating said transdermal delivery device to a temperature at which said active agent completely dissolves.
Clause 110. The method of clause 107, wherein said curing comprises heating said transdermal delivery device to a temperature about 20°C above the melting point of said active agent.
Clause 111. The method of clauses 109 and 110, wherein said curing comprises subjecting said device to oven infrared beams.
Clause 112. The method of clause 111, wherein said curing is performed for a duration ranging from about 1 second to about 10 minutes.
Clause 113. The method of clause 112, wherein said duration ranges from about 3 seconds to about 5 minutes.
Clause 114. A method of storing and protecting a transdermal delivery device having a backing layer, an adhesive matrix layer comprising a supersaturated concentration of at least one active agent substantially in amorphous form within said adhesive matrix, and a release liner said method comprising packaging said transdermal delivery device in a pouch.
Clause 115. The method of clause 114, wherein said pouch is comprised of paper, polymer film, metal foil or combinations thereof.
Clause 116. The method of clause 114, wherein said pouch is the same size as said release liner.
Clause 117. The method of clause 114, wherein said pouch is larger than said release liner.
Clause 118. The method of clause 114, wherein said pouch is about 0.1mm to about 20mm larger than said release liner.

## Claims

1. A transdermal delivery device comprising:
a) a backing layer,
b) an adhesive matrix layer comprising a supersaturated concentration of at least one active agent substantially in amorphous form within said adhesive matrix, and
c) a release liner,
wherein said active agent is selected from the group consisting of piroxicam, fentanyl, naltrexone, scopolamine, and a steroid.

2. The transdermal delivery device of claim 1, wherein said steroid is selected from the group consisting of estrogens, progestogens, testosterone, noregestrel, norethindrone acetate, medroxyprogesterone acetate, levonrgestrel, and norelgestromin.

3. The transdermal delivery device of any of the preceding claims, wherein said active agent is present in an amount of about 1% to about 20% by weight of said adhesive matrix.

4. The transdermal delivery device of any of the preceding claims, wherein a concentration of said active agent is from about 5% to about 5000% above the solubility of said active agent in said adhesive matrix.

5. The transdermal delivery device of any of the preceding claims, wherein said backing layer is selected from the group consisting of polyester films, polyethelene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.

6. The transdermal delivery device of any of the preceding claims, wherein said release liner is selected from the group consisting of polyester liners, polyurethane liners, polyester liners with a silicone coating, polyurethane liners with a silicone coating, polyester liners with a fluorosilicone coating, polyurethane liners with a fluorosilicone coating, silicon coated polyester liners, silicon coated polyurethane liners, polyester liners with a fluoropolymer coating, and polyurethane liners with a fluoropolymer coating.

7. The transdermal delivery device of any of the preceding claims, wherein at least one of said backing layer or said release liner is about 0.1mm to about 20mm larger than said adhesive matrix layer.

8. The transdermal delivery device of any of the preceding claims, wherein said adhesive matrix layer comprises an adhesive material selected from the group consisting of polyisobutylene, polysiloxane, acrylic adhesives, natural and synthetic rubber adhesives, and mixtures thereof, and wherein said adhesive material is present in an amount of from about 50% to about 99% by weight of said adhesive matrix layer.

9. The transdermal delivery device of any of the preceding claims, wherein said adhesive matrix layer further comprises one or more additives selected from the group consisting of penetration enhancers, crystal growth inhibitors, tackifiers, cohesive enhancers, plasticizers, and antioxidants, wherein said one or more additives are present in an amount of from about 1% to about 50% by weight of said adhesive matrix layer.

10. The transdermal delivery device of any of the preceding claims, wherein said adhesive matrix layer further comprises one or more cohesive enhancers selected from the group consisting of colloidal silicone dioxide, zinc oxide, polyvinylpyrrolidone, polyvinylpyrrolidone-co-vinylacetate, acrylate copolymers, crosspovidone, bentonites, clays, ethyl cellulose and mixtures thereof.

11. The transdermal delivery device of any of the preceding claims, wherein said adhesive matrix layer further comprises one or more flux enhancers selected from the group consisting of propylene glycol, butylene glycol, dipropylene glycol, diethylene glycol, propyl palmitate, isopropyl palmitate, propyl myristate, pendadecanol, octadecanol, propylene glycol monoesters, polyethylene glycol monoesters and glycerol monoesters.

12. The transdermal delivery device of any of the preceding claims, further comprising a drug release regulating membrane layer and a reservoir layer, wherein at least one of said drug release regulating membrane layer and said reservoir layer contains said active agent.

13. The transdermal delivery device of any of the preceding claims, further comprising an overlay layer in communication with said backing layer.

14. A method of preparing an adhesive matrix containing at least one active agent that is supersaturated and present in amorphous form comprising:
a) dissolving said at least one active agent and an adhesive material in a solvent in an amount so as to provide said at least one active agent at a subsaturated concentration in an adhesive matrix solution,
b) casting said subsaturated active agent in said adhesive matrix solution to one of a release liner and a backing layer,
c) removing said solvent at a temperature which is at, below, or above the melting point of said at least one active agent to form a dry adhesive matrix in which said at least one active agent is in a supersaturated concentration, and
d) laminating the other of said release liner and said backing film to said supersaturated active agent in said dry adhesive matrix, so that said supersaturated active agent in said dry adhesive matrix is between said release liner and said backing layer,
wherein said active agent is selected from the group consisting of piroxicam, fentanyl, naltrexone, scopolamine and a steroid, wherein said active agent is present in an amount of from about 0.1% to about 50% by weight of said adhesive matrix layer.

15. The method of claim 14, wherein said supersaturated active agent in said adhesive matrix further comprises one or more additives which are dispersed as liquid or solid particles in said dry adhesive matrix, said one or more additives are selected from the group consisting of penetration enhancers, crystal growth inhibitors, tackifiers, cohesive enhancers, plasticizers, and antioxidants, and wherein said one or more additives are present in an amount of from about 1% to about 50% by weight of said adhesive matrix.

16. A method of preparing an adhesive matrix containing at least one active agent that is supersaturated and present in amorphous form comprising:
a) admixing said at least one active agent with an adhesive matrix at a supersaturated concentration,
b) heating said supersaturated concentration of said at least one active agent in said adhesive matrix to a temperature which allows said at least one active agent to be completely dissolved and uniformly dispersed in said adhesive matrix to create a hot melt,
c) casting said hot melt to one of a release liner and a backing layer, at a predetermined temperature, and
d) laminating the other of said release liner and said backing layer to said hot melt, so that said hot melt is between said release liner and said backing layer,
wherein said active agent is selected from the group consisting of piroxicam, fentanyl, naltrexone, scopolamine and a steroid, wherein said active agent is present in an amount of from about 0.1% to about 50% by weight of said adhesive matrix layer.

17. The method of claim 16, wherein said supersaturated active agent in said adhesive matrix further comprises one or more additives which are dispersed as liquid or solid particles in said dry adhesive matrix, said one or more additives are selected from the group consisting of penetration enhancers, crystal growth inhibitors, tackifiers, cohesive enhancers, plasticizers, and antioxidants, and wherein said one or more additives are present in an amount of from about 1% to about 50% by weight of said adhesive matrix.
